# EUROPEAN PATENT APPLICATION

(11) **EP 2 034 018 A1**
(43) Date of publication of application: **11.03.2009**
(21) Application number: 07767621.1
(22) Date of filing: 26.06.2007
(51) Int. Cl.: C12N 15/09, A01K 67/027, A61K 45/00, A61P 3/04, A61P 3/10, A61P 43/00, C12N 5/10

(54) **GENETICALLY MODIFIED ANIMAL AND USE THEREOF**

(30) Priority: 27.06.2006 JP 2006176978
(71) Applicant: Takeda Pharmaceutical Company Limited, Osaka 541-0045 (JP)
(72) Inventor: TAKETOMI, Shigehisa, Hyogo 662-0095 (JP); NISHIDA, Mayumi, Osaka-shi, Osaka 532-8686 (JP)
(74) Representative: Keller, Günter
(86) International application number: PCT/JP2007/062815
(87) International publication number: WO 2008/001778

(57) **Abstract**

The present invention provides a non-human mammal deficient in the expression of the SLC-1 gene, having the characteristics of (1) a lower blood insulin level in glucose tolerance test, (2) increased insulin sensitivity, (3) higher resistance to obesity even on high fat diet, (4) a smaller white fat cell size, and (5) accentuated lipolysis, compared with the corresponding wild-type animal, or a portion of the body thereof. Also provided is an obesity and/or type II diabetes model non-human mammal that is deficient in the expression of the SLC-1 gene, having the characteristics of (1) elevated expression of adiponectin, (2) delayed onset of hyperglycemia, (3) a lower blood glycohemoglobin level, and (4) accentuated energy consumption, compared with the corresponding obesity and/or type II diabetes model non-human mammal wherein the expression of the gene is normal, or a portion of the body thereof.

SEQUENCE LISTING

<110>  Takeda Pharmaceutical Company Limited

<120>  Genetically Modified Animal and Use Thereof

<130>  091091

<150>  JP 2006-176978
<151>  2006-06-27

<160>  7

<170>  PatentIn version 3.3

<210>  1
<211>  20
<212>  DNA
<213>  Artificial

<220>
<223>  Primer

<400>  1
ctaaagcgca tgctccagac                                                    20


<210>  2
<211>  20
<212>  DNA
<213>  Artificial

<220>
<223>  Primer

<400>  2
atatcaggta ttagagtgac                                                    20


<210>  3
<211>  20
<212>  DNA
<213>  Artificial

<220>
<223>  Primer

<400>  3
ccgcttccat tgctcagcgg                                                    20


<210>  4
<211>  20
<212>  DNA
<213>  Artificial

<220>
<223>  Primer

<400>  4
gcttggtgct gtcggtgaag                                                    20


<210>  5
<211>  18
<212>  DNA
<213>  Artificial

<220>
<223>  Primer

<400>  5
tattctgtca aggggatc                                                      18

<210>  6
<211>  20
<212>  DNA
<213>  Artificial

<220>
<223>  Primer

<400>  6
cctcgcacaa ggagtgtctc                                                    20


<210>  7
<211>  20
<212>  DNA
<213>  Artificial

<220>
<223>  Primer

<400>  7
taatgaacga gagagcccac                                                    20

## Description

### Technical Field

The present invention relates to a non-human mammal deficient in the expression of the SLC-1 gene and an obesity and/or type II diabetes model non-human mammal that is deficient in the expression of the SLC-1 gene. The present invention also relates to a use of SLC-1 antagonizing action for promoting adiponectin production.

### (Background of the Invention)

Currently, melanin-concentrating hormone (MCH) is attracting worldwide attention as the most promising target of anti-obesity drugs. MCH, a circular peptide involved in the concentration of somatic pigments, first discovered in the salmon pituitary, is remarkably localized in the lateral field of the hypothalamus in mammals, and MCH-positive neurons are projected widely in the brain. Because mice deficient in the MCH gene exhibited decreased food intake and emaciation, and also because the phenotype of obesity was observed as a result of increased food intake and decreased energy consumption in mice with overexpression of the MCH gene, the involvement of MCH in overeating and obesity was strongly suggested. However, because mice having the MCH gene thereof manipulated were concurrently deficient in the neuropeptide GE, neuropeptide EI and the like, which are encoded on the same gene as MCH, to clarify the action of MCH, it was necessary to create and analyze mice deficient in the receptor thereof.

In humans, there have been reported two kinds of MCH receptors, i.e., somatostatin-like receptor 1: SLC-1 (MCHR1) and SLT (MCHR2), which are G protein coupled receptors (GPCRs); however, in rodents, SLT does not exist, but SLC-1 only is expressed. Expression of these two kinds of receptors is observed mainly in the brain; in humans, SLC-1, compared with SLT, is reportedly expressed at higher levels in the hypothalamus and at lower levels in the cerebral cortex, hippocampus and the like. Therefore, regarding the broad range of actions of MCH, such as eating, memory, reproduction, and behavior, it is suggested that SLC-1 may be involved in eating, and that SLT may be involved in emotion, memory and the like.

To date, SLC-1-deficient mice have been prepared by a group of Marsh et al. (non-patent document 1) and a group of Chen et al. (non-patent document 2), and have been reported to exhibit the phenotypes of body weight gain suppression and body fat mass reduction, despite overeating, due to increased energy consumption that accompanies increased spontaneous movement and oxygen consumption. Furthermore, the double deficient mice obtained by mating SLC-1-deficient mice and obesity/diabetes model ob/ob mice (deficient in leptin), compared with ob/ob mice, have been reported to be not different in terms of body weight, food intake, or energy consumption, but to exhibit suppressed blood glucose elevations, decreased insulin levels, decreased body fat, increased movement, and altered body temperature regulation in oral glucose tolerance tests (non-patent document 3).
[Non-patent document 1] Marsh, D.J. et al., Proceedings of the National Academy of Sciences, USA (Proc. Natl. Acad. Sci. USA), (US), 2002, vol. 99, p. 3240-3245
[Non-patent document 2] Chen, Y. et al., Endocrinology (Endocrinol.), (US), 2002, vol. 143, p. 2469-2477
[Non-patent document 3] Bjursell, M. et al., Diabetes, (US), 2006, vol. 55, p. 725-733

### [Disclosure of the Invention]

### [Problems to Be Solved by the Invention]

It is an object of the present invention to elucidate the functions of SLC-1 in living organisms by preparing and analyzing animals deficient in the expression of the SLC-1 gene, and to provide a novel and effective anti-obesity drug, antidiabetic drug and the like by reflecting the findings thus obtained in drug discovery research.

### [Means of Solving the Problems]

The present inventors, with the aim of accomplishing the object described above, created SLC-1 knockout (KO) mice having the receptor function destroyed by deleting the 7 transmembrane region of the mouse SLC-1 gene, and analyzed the phenotypes thereof. As a result, the KO mice, compared with wild-type mice, exhibited characteristics such as improved glucose tolerance, increased insulin sensitivity, smaller fat cell size, and accentuated lipolysis.
Furthermore, the present inventors performed a phenotype analysis on the mice obtained by crossing the KO mice and KKA^{y} mice, which are obesity/type II diabetes model mice, and, as a result, found that the plasma adiponectin level is elevated in the mice, compared with the KKA^{y} mice. This strongly suggests that SLC-1 antagonists may be effective in promoting adiponectin production in humans with obesity (particularly visceral fat type obesity).
The present inventors conducted further investigations based on these findings, and developed the present invention.

Accordingly, the present invention provides the following:
[1] A non-human mammal deficient in the expression of the SLC-1 gene, having the following characteristics:
   (1) a lower blood insulin level in glucose tolerance test,
   (2) increased insulin sensitivity,
   (3) higher resistance to obesity even on high fat diet,
   (4) a smaller white fat cell size, and
   (5) accentuated lipolysis
      compared with the corresponding wild-type animal, or a portion of the body thereof;
[2] the animal according to [1] above, further having the following characteristics:
   (i) accentuated spontaneous movement and oxygen consumption,
   (ii) decreased body fat, and
   (iii) a decreased plasma leptin level
      compared with the corresponding wild-type animal, or a portion of the body thereof;
[3] the animal according to [1] above, wherein the non-human mammal is a mouse or a rat, or a portion of the body thereof;
[4] an obesity and/or type II diabetes model non-human mammal that is deficient in the expression of the SLC-1 gene, having the following characteristics:
   (1) elevated adiponectin expression,
   (2) delayed onset of hyperglycemia,
   (3) a lower blood glycohemoglobin level, and
   (4) accentuated energy consumption
      compared with the corresponding obesity and/or type II diabetes model non-human mammal wherein the expression of the gene is normal, or a portion of the body thereof;
[5] the animal according to [4] above, further having the following characteristics:
   (i) increased oxygen consumption, and
   (ii) a decreased blood corticosterone level
      compared with the corresponding obesity and/or type II diabetes model non-human mammal wherein the expression of the SLC-1 gene is normal, or a portion of the body thereof;
[6] the animal according to [4] above, wherein the non-human mammal is a mouse or a rat, or a portion of the body thereof;
[7] the animal according to [6] above, wherein the obesity and/or type II diabetes model non-human mammal is a KKA^{y} mouse, or a portion of the body thereof;
[8] a promoter of adiponectin production comprising an SLC-1 antagonist;
[9] the agent according to [8] above, which is administered to a patient with metabolic syndrome or arteriosclerotic disease accompanied by a decreased adiponectin level;
[10] a method of promoting adiponectin production, comprising antagonistically inhibiting SLC-1;
[11] a use of an SLC-1 antagonist for producing a promoter of adiponectin production.

### [Effect of the Invention]

Because the non-human mammal deficient in the expression of the SLC-1 gene of the present invention exhibits the phenotypes of accentuated lipolysis, smaller fat cell size, increased insulin sensitivity, improved glucose tolerance and the like, it is useful in elucidating the functions of SLC-1 in vivo. By mating with the mouse, obesity/diabetes model mice can be made to be deficient in the SLC-1 gene, whereby the efficacy of an SLC-1 antagonist as an anti-obesity drug and antidiabetic drug can be testified.
Furthermore, according to the present invention, SLC-1 deficiency in obesity/diabetes mice results in an elevated adiponectin level; therefore, SLC-1 antagonists are useful in ameliorating the reduction in adiponectin content that accompanies visceral fat accumulation, which is thought to be an important upstream factor of metabolic syndrome.

### [Brief Description of the Drawings]

[FIG. 1] (A) shows the targeting vector used to prepare mice deficient in the SLC-1 gene (-/-) and the mode of homologous recombination. The exon 2 of the SLC-1 gene was replaced with the neomycin resistance gene to destroy the function thereof. (B) shows the expression of the SLC-1 gene in the whole brains of SLC-1 homo-deficient (-/-), hetero-deficient (+/-), and wild (+/+) mice. In the SLC-1(-/-) mouse, the expression of the SLC-1 gene has been lost.
[FIG. 2] (A) shows growth curves for SLC-1(-/-) mice. The animals were reared on an ordinary diet or a high fat diet for 15 weeks from 5 weeks of age. (B) shows daily food intake per unit body weight measured at 8 weeks of age. The data are shown as mean ± standard deviation.
[FIG. 3] Shows spontaneous movement (A) and oxygen consumption (B) for SLC-1(-/-) mice. The animals were reared on an ordinary diet or a high fat diet from 5 weeks of age. Spontaneous movement is shown as mean ± standard deviation for individuals at 12 weeks of age (n=16). Oxygen consumption is shown as mean ± standard deviation for individuals at 12 to 13 weeks of age (n=20).
[FIG. 4] Shows a glucose tolerance test (A) and an insulin tolerance test (B), and an insulin resistance test in peripheral tissue (C) on SLC-1(-/-) mice. After loading an ordinary diet or a high fat diet from 5 weeks of age to the week of age at which each test was performed, SLC-1(+/+) or (-/-) mice were fasted for 20 hours, and the glucose tolerance, insulin tolerance, or insulin resistance test was performed. The data are shown as mean ± standard deviation.
[FIG. 5] Shows lipolysis in SLC-1(-/-) mice. This was performed using perigenital white adipose tissue from mice reared on an ordinary diet from 5 weeks of age. The data are shown as mean ± standard deviation.
[FIG. 6] Shows growth curves (A) and daily food intake per unit body weight (B) for KKA^{y} mouse and KK mouse hybrid groups. Body weights were measured for 16 weeks from 2 weeks of age, and food intake was measured at 6 weeks of age. The data are shown as mean ± standard deviation.
[FIG. 7] Shows plasma parameters for KKA^{y} mouse and KK mouse hybrid groups. Plasma glucose level (A), plasma triglyceride level (B), plasma insulin level (C), plasma adiponectin level (D), plasma leptin level (E), hemoglobin (Hb) A1c level (F), plasma nonesterified fatty acid (NEFA) level (G), plasma corticosterone level (H), and plasma total T4 level (I) were measured. The data are shown as mean ± standard deviation.
[FIG. 8] Body fat percentages in KKA^{y} mouse and KK mouse hybrid groups were measured. The measurements were taken at 17 weeks of age. The data are shown as mean ± standard deviation.
[FIG. 9] Shows oxygen consumption (A) and respiratory quotient (B) at 13 to 14 weeks of age, and cumulative spontaneous movement at 7 to 9 weeks of age (C) for KKA^{y} mouse and KK mouse hybrid groups. The data are shown as mean ± standard deviation.
[FIG. 10] Shows plasma glucose level (A) and plasma insulin level (B) in a glucose tolerance test on KKA^{y} mouse and KK mouse hybrid groups at 16 weeks of age. The data are shown as mean ± standard deviation.
[FIG. 11] Shows gene expression levels in KKA^{y} mouse and KK mouse hybrid groups at 9 weeks of age. Changes in the diencephalon (A), perigenital white adipose (B), liver (C), and skeletal muscle (D) were examined.

### (Detailed Description of the Invention)

The present invention provides a non-human mammal deficient in the expression of the SLC-1 gene.

A non-human mammal deficient in the expression of the SLC-1 gene means a non-human mammal having the expression of endogenous SLC-1 inactivated therein, including SLC-1 KO animals prepared from an ES cell having the SLC-1 gene knocked out (KO) therein, as well as knockdown (KD) animals having the expression of the SLC-1 gene inactivated by antisense or RNAi technology therein, and the like. Here, "knocked out (KO)" means that the production of complete mRNA is prevented by destroying or removing the endogenous gene, whereas "knocked down (KD)" means that translation from mRNA into protein is inhibited to inactivate the expression of the endogenous gene. Hereinafter, the SLC-1 gene KO/KD animal of the present invention is sometimes simply referred to as "the KO/KD animal of the present invention".

"A non-human mammal" that can be a subject of the present invention is not particularly limited, as long as it is a non-human mammal for which a transgenic system has been established; examples include mice, rats, bovines, monkeys, pigs, sheep, goat, rabbits, dogs, cats, guinea pigs, hamsters and the like. Mice, rats, rabbits, dogs, cats, guinea pigs, hamsters and the like are preferable; in particular, from the viewpoint of the preparation of disease model animals, rodents, which have relatively short periods of ontogeny and life cycles, and which are easy to propagate, are more preferable; particularly, mice (for example, C57BL/6 strain, BALB/c strain, DBA2 strain and the like as pure strains, B6C3F₁ strain, BDF₁ strain, B6D2F₁ strain, ICR strain and the like as hybrid strains) and rats (for example, Wistar, SD and the like) are preferable.
In addition to mammals, birds such as chickens can be used for the same purpose as that of "non-human mammals" being subjects of the present invention.

As a specific means for knocking out the SLC-1 gene, there can be preferably used a method comprising isolating the SLC-1 gene (genomic DNA) derived from the subject non-human mammal by a conventional method, and integrating a DNA strand having a DNA sequence constructed to consequently inactivate the gene by, for example, (1) destroying the function of the exon or promoter by inserting another DNA fragment (for example, drug resistance gene, reporter gene and the like) into the exon portion or promoter region, or (2) cutting out the entire or a portion of the SLC-1 gene using the Cre-loxP system or Flp-frt system to delete the gene, or (3) inserting a stop codon into the protein coding region to prevent the translation into complete protein, or (4) inserting a DNA sequence that stops the transcription of the gene (for example, polyA addition signal and the like) into the transcription region to prevent the synthesis of complete mRNA, (hereinafter, abbreviated as targeting vector), at the SLC-1 gene locus of the subject non-human mammal by homologous recombination, and the like.

The homologous recombinant can be acquired by, for example, introducing the above-described targeting vector into an embryonic stem cell (ES cell).
An ES cell refers to a cell derived from an inner cell mass (ICM) of a fertilized egg in the blastocyst stage, and can be cultivated and maintained while keeping the undifferentiated state in vitro. ICM cells are destined to form the embryo body, being stem cells on which all tissues, including germ cells, are based. The ES cell used may be of an established cell line, or of a cell line newly established in accordance with the method of Evans and Kaufman (Nature, vol.292, p.154, 1981). For example, in the case of mouse ES cells, ES cells derived from a 129 mouse strain are currently generally used, but the immunological background thereof is unclear; for the purposes of acquiring ES cells of a pure strain instead thereof with an immunologically clear genetic background and the like, an ES cell established from a C57BL/6 mouse or from a BDF₁ mouse (F₁ of C57BL/6 and DBA/2), wherein the small number of ova collectable from C57BL/6 has been improved by crossing with DBA/2, and the like can also be used suitably. In addition to being advantageous in that the number of ova collectable is high, and that the ova are robust, BDF₁ mice have the C57BL/6 mouse as the background thereof; therefore, ES cells derived therefrom can be used advantageously in that, when preparing a disease model mouse, the genetic background can be replaced with that of the C57BL/6 mouse by back-crossing with a C57BL/6 mouse.

ES cells can be prepared by, for example, as described below. When a blastocystic embryo is collected from the uterus of a female non-human mammal after mating [for example, when a mouse (preferably a mouse of an inbred strain such as C57BL/6J(B6), F₁ of B6 and another inbred strain, and the like) is used, a female mouse at about 8 to about 10 week-old (about 3.5 days of gestation) mated with a male mouse at about 2 month-old or more is preferably used] (or an early embryo in the morula stage or before is collected from the oviduct, after which it may be cultured in a medium for embryo culture as described above until the blastocyst stage), and cultured on a layer of appropriate feeder cells (for example, in the case of a mouse, primary fibroblasts prepared from a fetal mouse, commonly known STO fibroblast line and the like), some cells of the blastocyst gather to form an ICM that will differentiate into an embryo. This inner cell mass is trypsinized to dissociate single cells, and while maintaining an appropriate cell density and making medium exchanges, dissociation and passage are repeated, whereby ES cells are obtained.

Although both male and female ES cells can be used, male ES cells are usually more convenient in preparing a germline chimera. Also for the sake of saving painstaking labor for cultivation, it is desirable that sex identification be performed as early as possible. An example of the method of identifying the sex of an ES cell is a method comprising amplifying and detecting a gene in the sex determining region on Y chromosome by PCR. Using this method, about 1 colony of ES cells (about 50 cells) is sufficient, compared with the conventional method, which requires about 10⁶ cells for karyotype analysis, so that primary selection of ES cells in early stages of cultivation can be performed by sex identification, thus making early selection of male cells possible, whereby labor in early stages of cultivation can be reduced significantly.
Secondary selection can be performed by, for example, confirming chromosome numbers by the G-banding method, and the like. It is desirable that the chromosome number of the ES cell obtained be 100% of the normal number.

The ES cell line thus obtained needs to be subcultured carefully to maintain the nature of undifferentiated stem cells. For example, the ES cell line is cultured by, for example, a method comprising culturing on appropriate feeder cells, like STO fibroblasts, in the presence of LIF (1 to 10,000 U/ml), known as a differentiation suppressing factor, in a gaseous carbon dioxide incubator (preferably, 5% gaseous carbon dioxide/95% air or 5% oxygen/5% gaseous carbon dioxide/90% air) at about 37°C, and the like; upon passage, for example, the ES cell line is treated with trypsin/EDTA solution (usually 0.001 to 0.5% trypsin/0.1 to 5 mM EDTA, preferably about 0.1% trypsin/1 mM EDTA) to obtain single cells, which are sown onto freshly prepared feeder cells, and the like. This passage is normally performed every 1 to 3 days, during which the cells were examined; if a morphologically abnormal cell is found, it is desirable that the cultured cells be discarded.

ES cells can be differentiated into a wide variety of types of cell, including parietal muscle, visceral muscles, and cardiac muscle, by monolayer culture until the reach of a high density, or suspension culture until the formation of cell aggregates, under appropriate conditions [M.J. Evans and M.H. Kaufman, Nature vol.292, p.154, 1981; G.R. Martin, Proceedings of the National Academy of Sciences, USA (Proc. Natl. Acad. Sci. U.S.A.), vol.78, p.7634, 1981; T. C. Doetschman et al., Journal of Embryology and Experimental Morphology, vol.87, p.27, 1985]; the SLC-1 non-human mammal deficient in the expression of the gene, according to the present invention, obtained by differentiating an ES cell incorporating targeting vector, are useful in cell biological investigations of SLC-1 in vitro.

For example, if a targeting vector is designed to destroy the function of an exon or promoter by inserting another DNA fragment into the exon portion or promoter region of the SLC-1 gene, the vector can assume, for example, the constitution shown below.

First, to ensure that another DNA fragment is inserted into the exon or promoter portion of the SLC-1 gene by homologous recombination, the targeting vector need to contain sequences homologous to the respective target sites (5' arm and 3' arm) upstream of the 5' and downstream of the 3' in the other DNA fragment (for example, in an Example below, to destroy exon 2, the targeting vector contains a sequence homologous to the region spanning from 5' regulatory region to intron 1 of the SLC-1 gene upstream of the 5' of the other DNA fragment inserted, and a sequence homologous to the region spanning from a portion of the exon 2 to a portion of the intron 2 downstream of the 3').

Although the other DNA fragment inserted is not particularly limited, it is possible to select ES cells having a targeting vector integrated in a chromosome thereof with drug resistance or reporter activity as the index, by using a drug resistance gene or a reporter gene. Here, examples of the drug resistance gene and examples of the reporter gene include, but are not limited to, the neomycin phosphotransferase II (nptII) gene, the hygromycin phosphotransferase (hpt) gene and the like, and the β-galactosidase (lacZ) gene, the chloramphenicol acetyltransferase (cat) gene and the like, respectively.

The drug resistance or reporter gene is preferably under the control of an optionally chosen promoter capable of functioning in mammalian cells. For example, virus promoters such as the SV40 early promoter, cytomegalovirus (CMV) long terminal repeat (LTR), Rous sarcoma virus (RSV) LTR, mouse leukemia virus (MoMuLV) LTR, and adenovirus (AdV)-derived early promoter, and promoters for mammalian constitutive protein genes such as the β-actin gene promoter, PGK gene promoter, and transferrin gene promoter and the like can be mentioned. However, if the drug resistance or reporter gene is inserted into the SLC-1 gene so that it is placed under the control of an endogenous promoter of the SLC-1 gene, a promoter that controls the transcription of the gene need not be present in the targeting vector.

The targeting vector preferably has a sequence that terminates the transcription of mRNA from the gene (polyadenylation (polyA) signal, also called terminator) downstream of the drug resistance or reporter gene; for example, terminator sequences derived from virus genes, or from various mammal or bird genes, can be used. Preferably, an SV40 terminator and the like are used.

Usually, gene recombination in a mammal occurs mostly non-homologously; the introduced DNA is randomly inserted at an optionally chosen position on the chromosome. Therefore, it is not possible to efficiently select only those clones targeted to the endogenous SLC-1 gene targeted by homologous recombination by selection based on the detection of the expression of a drug resistance or reporter gene and the like (positive selection); it is necessary to confirm the site of integration by Southern hybridization or PCR for all the clones selected. Hence, provided that, for example, the herpes simplex virus-derived thymidine kinase (HSV-tk) gene, which confers gancyclovir susceptibility, is joined outside the region homologous to the target sequence of the targeting vector, the cells having the vector inserted randomly thereinto cannot grow in a gancyclovir-containing medium because they have the HSV-tk gene, whereas the cells targeted to the endogenous SLC-1 locus by homologous recombination become resistant to gancyclovir and are selected because they do not have the HSV-tk gene (negative selection). Alternatively, provided that the diphtheria toxin gene, for example, is joined in place of the HSV-tk gene, the cells having the vector inserted randomly thereinto die due to the toxin produced by themselves, so that a homologous recombinant can also be selected in the absence of a drug.

Although any of the calcium phosphate co-precipitation method, electroporation method, lipofection method, retrovirus infection method, aggregation method, microinjection method, gene gun (particle gun) method, DEAE-dextran method and the like can be used for targeting vector introduction into ES cells, the electroporation method is generally chosen because of the ease of treatment of a large number of cells and the like, since gene recombination in a mammal occurs mostly non-homologously so that the frequency of obtainment of homologous recombinants is low, as described above. For the electroporation, ordinary conditions used for gene introduction into animal cells may be used as is; for example, the electroporation can be performed by trypsinizing ES cells in the logarithmic growth phase to disperse them as single cells, suspending the cells in a medium to obtain a density of 10⁶ to 10⁸ cells/ml, transferring the cells to a cuvette, adding 10 to 100 µg of a targeting vector, and applying an electric pulse of 200 to 600 V/cm.

ES cells having the targeting vector integrated therein can be determined by screening chromosome DNA separated and extracted from a colony obtained by culturing the single cells on feeder cells, by Southern hybridization or PCR; if a drug resistance gene or a reporter gene is used as the other DNA fragment, it is possible to select a transformant at the cellular stage with the expression thereof as the index. For example, if a vector comprising the nptII gene as the marker gene for positive selection is used, ES cells after gene introduction treatment are cultured in a medium containing a neomycin-series antibiotic such as G418, and the resulting resistant colony is selected as a candidate for a transformant. If a vector comprising the HSV-tk gene is used as the marker gene for negative selection, the ES cells are cultured in a medium containing ganciclovir, and the resulting resistant colony is selected as a candidate for a homologous recombinant. The colonies obtained are transferred to respective culture plates, and trypsinization and medium exchanges are repeated, after which a portion is reserved for cultivation, and the remainder is subjected to PCR or Southern hybridization to confirm the presence of the introduced DNA.

When an ES cell confirmed to have the introduced DNA integrated therein is returned to an embryo derived from a non-human mammal of the same species, the ES cell gets integrated into the ICM of the host embryo to form a chimeric embryo. This is transplanted into a recipient mother (embryo recipient female) and allowed to continue development, whereby a chimeric KO animal is obtained. If the ES cell contributes to the formation of a primordial germ cell that will differentiate into an egg or spermatozoon in the chimeric animal, a germline chimera will be obtained; by mating this, a KO animal having deficiency of the SLC-1 gene maintained genetically therein can be prepared.

For preparing a chimeric embryo, there are a method wherein early embryos up to the morula stage are adhered and aggregated together (aggregation chimera method) and a method wherein a cell is micro-injected into a blastocoel cavity of a blastocyst (injection chimera method). Although the latter has traditionally been widely conducted in the preparation of a chimeric embryo using an ES cell, a method wherein an aggregation chimera is created by injecting an ES cell into the zona pellucida of an 8-cell stage embryo, and a method wherein an aggregation chimera is created by co-culturing and aggregating an ES cell mass and an 8-cell stage embryo deprived of the zona pellucida, as a method which does not require a micromanipulator and which can be easily operated, have recently been conducted.

In all cases, a host embryo can be collected from a non-human mammal that can be used as a female for egg collection in gene introduction into a fertilized egg as below mentioned in the same manner; for example, in the case of a mouse, to make it possible to determine the percent contribution of ES cells to the formation of a chimera mouse by coat color, it is preferable that the host embryo be collected from a mouse of a strain showing a coat color different from that of the strain from which the ES cell is derived. For example, in the case of an ES cell derived from a 129 mouse strain (coat color: agouti), a C57BL/6 mouse (coat color: black) or an ICR mouse (coat color: albino) is used as the female for egg collection; in the case of an ES cell derived from a C57BL/6 or DBF₁ mouse (coat color: black) or from a TT2 cell (derived from F₁ (coat color: agouti) of C57BL/6 and CBA), an ICR mouse or a BALB/c mouse (coat color: albino) can be used as the female for egg collection.

Because the germline chimera formation capacity depends largely on the combination of an ES cell and a host embryo, it is more preferable that a combination showing a high germline chimera formation capacity be chosen. For example, in the case of a mouse, it is preferable to use a host embryo derived from the C57BL/6 strain and the like for ES cells derived from the 129 strain, and to use a host embryo derived from the BALB/c strain and the like for ES cells derived from the C57BL/6 strain.

It is preferable that the female mouse for egg collection be about 4 to about 6 week-old, and that the male mouse for mating be of the same strain at about 2 to about 8 month-old. Although the mating may be by natural mating, it is preferably performed after administering gonadotropic hormones (follicle-stimulating hormone, then luteinizing hormone) to induce overovulation.

In the case of the blastocyst injection method, a blastocystic embryo (for example, in the case of a mouse, at about 3.5 days after mating) is collected from the uterus of a female for egg collection (or an early embryo in the morula stage or before, after being collected from the oviduct, may be cultured in a medium (below-mentioned) for embryo culture until the blastocyst stage), and ES cells (about 10 to about 15 cells) having a targeting vector introduced thereinto are injected into a blastocoel cavity of the blastocyst using a micromanipulator, after which the embryos are transplanted into the uterus of a pseudopregnant embryo recipient female non-human mammal. As the embryo recipient female non-human mammal, a non-human mammal that can be used as an embryo recipient female in gene introduction into a fertilized egg can be used in the same manner.

In the case of the co-culture method, 8-cell stage embryos and morulas (for example, in the case of a mouse, about 2.5 days after mating) are collected from the oviduct and uterus of a female for egg collection (or an early embryo in the 8-cell stage or before, after being collected from the oviduct, may be cultured in a medium (below-mentioned) for embryo culture until the 8-cell stage or morula stage), and the zona pellucida is lysed in acidic Tyrode's solution, after which an ES cell mass incorporating a targeting vector (number of cells: about 10 to about 15 cells) is placed in a microdrop of a medium for embryo culture overlaid with mineral oil, the above-described 8-cell stage embryo or morula (preferably 2 embryos) is further placed, and they are co-cultured overnight. The morula or blastocyst obtained is transplanted to the uterus of an embryo recipient female non-human mammal as described above.

If the transplanted embryo implants successfully and the embryo recipient female becomes pregnant, chimeric non-human mammal pups will be obtained by natural delivery or caesarean section. Embryo recipient females that have delivered spontaneously are allowed to continue suckling; if the pups are delivered by caesarean section, the pups can be suckled by a separately provided female for suckling (a female non-human mammal with usual mating and delivery).

For the selection of a germline chimera, if the sex of the ES cell has already been determined, a chimera mouse of the same sex as the ES cell first is selected (usually, a male chimera mouse is chosen since a male ES cell is used), and then a chimera mouse showing a high ES cell contribution rate (for example, 50% or more) is selected on the basis of phenotypes such as coat color. For example, in the case of a chimera mouse obtained from a chimera embryo between a D3 cell, which is a male ES cell derived from a 129 mouse strain, and a host embryo derived from a C57BL/6 mouse, it is preferable that a male mouse showing a high percentage of the agouti coat color be selected. Whether or not the selected chimera non-human mammal is a germline chimera can be determined on the basis of the phenotypes of the F₁ animal obtained by crossing with an appropriate strain of the same animal species. For example, in the case of the above-described chimera mouse, agouti is dominant over black; therefore, when the male mouse is crossed with a female C57BL/6 mouse, the coat color of the F₁ obtained is agouti if the selected male mouse is a germline chimera.

The thus-obtained germline chimera non-human mammal incorporating a targeting vector (founder) is usually obtained as a heterozygote having the SLC-1 gene only knocked out in either one of the homologous chromosomes. To obtain a homozygote having the SLC-1 gene knocked out in both homologous chromosomes, of the F₁ animals obtained as described above, siblings of heterozygotes may be crossed. Selection of heterozygotes can be determined by, for example, screening chromosome DNAs separated and extracted from the tail of an F₁ animal by Southern hybridization or PCR. 1/4 of the F₂ animals obtained will be homozygotes.

In another preferred embodiment with the use of a virus as the targeting vector, a method comprising infecting an ES cell of a non-human mammal with a virus comprising a DNA comprising a marker gene for positive selection inserted between the 5' and 3' arms, and a marker gene for negative selection outside the arms, can be mentioned (see, for example, Proceedings of the National Academy of Sciences, USA (Proc. Natl. Acad. Sci. USA), vol.99, No.4, pp. 2140-2145, 2002). For example, when retrovirus or lentivirus is used, cells are sown to an appropriate incubator such as a culture dish, a virus vector is added to the culture broth (if desired, polybrene may be co-present), the cells are cultured for 1 to 2 days, after which, cultivation is continued as described above, and cells having the vector integrated therein are selected.

Regarding specific means for knocking down the SLC-1 gene, a method comprising introducing a DNA that encodes an antisense RNA or siRNA (including shRNA) of SLC-1 using techniques of preparation of transgenic animals known per se, and allowing it in the subject non-human mammal cell and the like can be mentioned.

A DNA comprising a base sequence complementary to the target region of a desired polynucleotide, i.e., a DNA hybridizable with a desired polynucleotide, can be said to be "antisense" against the desired polynucleotide.
The antisense DNA having a base sequence complementary or substantially complementary to the base sequence of a polynucleotide that encodes SLC-1 or a portion thereof may be any antisense DNA, as long as it contains a base sequence complementary or substantially complementary to the base sequence of the polynucleotide that encodes SLC-1 or a portion thereof, and having an action to suppress the expression of the polynucleotide.

The base sequence substantially complementary to a polynucleotide that encodes SLC-1 is, for example, a base sequence having a homology of about 70% or more, preferably about 80% or more, more preferably about 90% or more, most preferably about 95% or more, to the base sequence of the complementary strand of the polynucleotide for the overlapping region. Base sequence homology herein can, for example, be calculated using the homology calculation algorithm NCBI BLAST (National Center for Biotechnology Information Basic Local Alignment Search Tool) under the following conditions (expect=10; gap allowed; filtering=ON; match score=1; mismatch score=-3).
Particularly, of the full base sequence of the complementary strand of the polynucleotide that encodes SLC-1, (a) in the case of an antisense DNA intended to inhibit the translation, an antisense DNA having a homology of about 70% or more, preferably about 80% or more, more preferably about 90% or more, most preferably about 95% or more, to the complementary strand of the base sequence of the portion that encodes the N-terminal part of SLC-1 protein (for example, a base sequence in the vicinity of the initiation codon and the like) is suitable, and (b) in the case of an antisense DNA intended to degrade RNA with RNaseH, an antisense DNA having a homology of about 70% or more, preferably about 80% or more, more preferably about 90% or more, most preferably about 95% or more, to the complementary strand of the full base sequence of the polynucleotide that encodes SLC-1 including the intron, is suitable.

Specifically, when the subject non-human mammal is a mouse, an antisense DNA comprising a base sequence complementary or substantially complementary to the base sequence registered under GenBank accession No. NM_145132 (VERSION: NM_145132.1, GI:21553072) or a portion thereof, preferably, an antisense DNA comprising a base sequence complementary to the base sequence or a portion thereof, and the like can be mentioned. When the subject non-human mammal is a rat, an antisense DNA comprising a base sequence complementary or substantially complementary to the base sequence registered under GenBank accession No. NM_031758 (VERSION: NM_031758.1, GI: 13929067) or a portion thereof, preferably, an antisense DNA comprising a base sequence complementary to the base sequence or a portion thereof, and the like can be mentioned.

An antisense DNA having a base sequence complementary or substantially complementary to the base sequence of a polynucleotide that encodes SLC-1 or a portion thereof (hereinafter, also referred to as "the antisense DNA of the present invention") can be designed and synthesized on the basis of base sequence information on a DNA that encodes cloned or determined SLC-1. Such antisense DNA is capable of inhibiting the replication or expression of the SLC-1 gene. Specifically, the antisense DNA of the present invention is capable of hybridizing with an RNA transcribed from the SLC-1 gene (mRNA or initial transcription product), and capable of inhibiting the synthesis (processing) or function (translation into protein) of mRNA.

The target region of the antisense DNA of the present invention is not particularly limited with respect to the length thereof, as long as the translation into SLC-1 protein is inhibited as a result of hybridization of the antisense DNA; the target region may be the entire sequence or a partial sequence of the mRNA that encodes the protein, and the length is about 10 bases for the shortest, and the entire sequence of the mRNA or initial transcription product for the longest. Specifically, the 5' end hairpin loop, 5' end 6-base-pair repeats, 5' end untranslated region, translation initiation codon, protein coding region, ORF translation stop codon, 3' end untranslated region, 3' end palindrome region, or 3' end hairpin loop of the SLC-1 gene may be chosen as a preferable target region of the antisense DNA, but any other region in the SLC-1 gene may also be chosen as the target. For example, the intron portion of the gene may also be the target region.
Furthermore, the antisense DNA of the present invention may be one that not only hybridizes with the mRNA or initial transcription product of SLC-1 to inhibit the translation into protein, but also is capable of binding to the SLC-1 gene being a double-stranded DNA to form a triple strand (triplex) and hence to inhibit the transcription to RNA. Alternatively, the antisense DNA of the present invention may be one that forms a DNA:RNA hybrid to induce the degradation by RNaseH.

A DNA that encodes a ribozyme capable of specifically cleaving the mRNA that encodes SLC-1 or the initial transcription product within the coding region (including the intron portion in the case of the initial transcription product) can also be encompassed in the antisense DNA of the present invention. One of the most versatile ribozymes is a self-splicing RNA found in infectious RNAs such as viroid and virusoid, and the hammerhead type, the hairpin type and the like are known. The hammerhead type exhibits enzyme activity with about 40 bases in length, and it is possible to specifically cleave the target mRNA by making several bases at both ends flanking to the hammerhead structure portion (about 10 bases in total) a sequence complementary to the desired cleavage site of the mRNA. Because this type of ribozyme has only RNA as the substrate, it offers an additional advantage of non-attack of genomic DNA. Provided that the SLC-1 mRNA assumes a double-stranded structure per se, the target sequence can be made to be single-stranded by using a hybrid ribozyme prepared by joining an RNA motif derived from a viral nucleic acid that can bind specifically to RNA helicase [Proc. Natl. Acad. Sci. USA, 98(10): 5572-5577 (2001)]. Furthermore, the ribozyme may be a hybrid ribozyme prepared by further joining a sequence modified from the tRNA to promote the translocation of the transcription product to cytoplasm [Nucleic Acids Res., 29(13): 2780-2788 (2001)].

Herein, a double-stranded DNA consisting of an oligo-RNA homologous to a partial sequence (including the intron portion in the case of the initial transcription product) in the coding region of the mRNA or initial transcription product of SLC-1 and a strand complementary thereto, what is called a short-chain interfering RNA (siRNA), can also be used to prepare the KD animal of the present invention. It had been known that so-called RNA interference (RNAi), which is a phenomenon that when siRNA is introduced into cells, an mRNA homologous to the RNA is degraded, occurs in nematodes, insects, plants and the like; since this phenomenon was confirmed to also occur in animal cells [Nature, 411(6836): 494-498 (2001)], siRNA has been widely utilized as an alternative technique to ribozymes. siRNA can be designed as appropriate on the basis of base sequence information of the mRNA being the target using commercially available software (e.g., RNAi Designer; Invitrogen).

The antisense oligo-DNA and ribozyme of the present invention can be prepared by determining the target sequence for the mRNA or initial transcription product on the basis of a cDNA sequence or genomic DNA sequence of SLC-1, and synthesizing a sequence complementary thereto using a commercially available DNA/RNA synthesizer (Applied Biosystems, Beckman, and the like). By inserting the synthesized antisense oligo-DNA or ribozyme downstream of the promoter in the expression vector, via an appropriate linker (adapter) sequence used as required, a DNA expression vector that encodes the antisense oligo-RNA or ribozyme can be prepared. Examples of expression vectors that can be used preferably here include plasmids amplified with *Escherichia coli, Bacillus subtilis,* or yeast, bacteriophages such as λ phage, retroviruses such as Moloney leukemia virus, animal or insect viruses such as lentivirus, adeno-associated virus, vaccinia virus and baculovirus, and the like. In particular, plasmids (preferably plasmids from *Escherichia coli, Bacillus subtilis,* or yeast, particularly plasmids from Escherichia coli) and animal viruses (preferably retrovirus, lentivirus) are preferable. Examples of promoters include virus promoter such as the SV40 early promoter, cytomegalovirus (CMV) long terminal repeat (LTR), Rous sarcoma virus (RSV) LTR, mouse leukemia virus (MoMuLV) LTR, and adenovirus (AdV) derived early promoter, and promoters for mammalian constitutive protein genes such as the β-actin gene promoter, PGK gene promoter, and transferrin gene promoter and the like.

A DNA expression vector that encodes a longer antisense RNA (for example, full-length complementary strand of SLC-1 mRNA and the like) can be prepared by inserting a SLC-1 cDNA, cloned by a conventional method, in the reverse direction, via an appropriate linker (adapter) sequence used as required, downstream of the promoter in the expression vector.

Meanwhile, a DNA that encodes siRNA can be prepared by separately synthesizing a DNA that encodes a sense strand and a DNA that encodes an antisense strand, and inserting them into an appropriate expression vector. As the siRNA expression vector, one having a Pol III system promoter such as U6 or H1 can be used. In this case, in the animal cell incorporating the vector, the sense strand and the antisense strand are transcribed and annealed to form siRNA. shRNA can be prepared by inserting a unit comprising a sense strand and an antisense strand separated by a length base allowing the formation of an appropriate loop structure (for example, about 15 to 25 bases) into an appropriate expression vector. As the shRNA expression vector, one having a Pol III system promoter such as U6 or H1 can be used. In this case, the shRNA transcribed in the animal cell incorporating the expression vector forms a loop by itself, and is then processed by an endogenous enzyme dicer and the like to form mature siRNA. Alternatively, it is also possible to achieve knockdown by RNAi by expressing a microRNA (miRNA) comprising the siRNA sequence being the target using a Pol II system promoter. In this case, by a promoter showing tissue-specific expression, tissue-specific knockdown is also possible.

For introducing an expression vector comprising a DNA that encodes an antisense RNA, siRNA, shRNA, or miRNA of SLC-1 into a cell, a method known *per* se is used as appropriate according to the target cell. For example, for introduction into an early embryo such as a fertilized egg, the microinjection method is used. For introduction into an ES cell, the calcium phosphate co-precipitation method, electroporation method, lipofection method, retrovirus infection method, aggregation method, microinjection method, particle gun method, DEAE-dextran method and the like can be used. Alternatively, when retrovirus, lentivirus and the like are used as the vector, it is sometimes possible to achieve gene introduction conveniently by adding the virus to an early embryo or an ES cell, and culturing the embryo or cell for 1 to 2 days to infect the cells with the virus. Regeneration of individuals from an ES cell (establishment of founder), passage (preparation of homozygotes) and the like can be performed as described above with respect to the KO animal of the present invention.

In a preferred embodiment, the expression vector comprising a DNA that encodes an antisense RNA, siRNA, shRNA, or miRNA of SLC-1 is introduced into an early embryo of a non-human mammal being the subject by microinjection.

An early embryo of the subject non-human mammal can be obtained by collecting an in vivo fertilized egg obtained by mating a male and female non-human mammal of the same species, or by in vitro fertilization of an ovum and spermatozoa collected from a female and male non-human mammal of the same species, respectively.
The age, rearing conditions and the like of the non-human mammal used vary depending on animal species; for example, when a mouse (preferably, a mouse of an inbred strain such as C57BL/6J(B6), F₁ of B6 and another inbred strain, and the like) is used, it is preferable that a female at about 4 to about 6 weeks of age and a male at about 2 to about 8 months of age be used, and that the mice be used after rearing with a bright phase of about 12 hours (for example, 7:00-19:00) for about 1 week.

Although the in vivo fertilization may be by spontaneous mating, a method is preferable comprising administering a gonadotropic hormone to a female non-human mammal to induce overovulation, and then mating the female with a male non-human mammal, for the purpose of adjusting the estrous cycle and obtaining a large number of early embryos from a single individual. For inducing ovulation in a female non-human mammal, for example, a method is preferable comprising administering a follicle-stimulating hormone (pregnant mare's serum gonadotropic hormone, generally abbreviated as PMSG), and then a luteinizing hormone (human chorionic gonadotropic hormone, generally abbreviated as hCG), by, for example, intraperitoneal injection and the like; preferable amounts and frequencies of administration of the hormones vary depending on the species of the non-human mammal. For example, when the non-human mammal is a mouse (preferably, a mouse of an inbred strain such as C57BL/6J(B6), F₁ of B6 and another inbred strain, and the like), a method is preferable comprising administering a follicle-stimulating hormone, then administering a luteinizing hormone about 48 hours later, and immediately mating the female mouse with a male mouse to obtain a fertilized egg, wherein the amount of the follicle-stimulating hormone administered is about 20 to about 50 IU/individual, preferably about 30 IU/individual, and the amount of the luteinizing hormone administered is about 0 to about 10 IU/individual, preferably about 5 IU/individual.

After elapse of a given time, a female non-human mammal confirmed to have copulated by vaginal plug examination and the like is laparotomized, a fertilized egg is removed from the oviduct, washed in a medium for embryo culture (e.g., M16 medium, modified Whitten medium, BWW medium, M2 medium, WM-HEPES medium, BWW-HEPES medium and the like) to remove cumulus oophorus cells, and cultured in 5% gaseous carbon dioxide/95% atmosphere by the microdrop culture method and the like until DNA microinjection. If microinjection is not immediately performed, the fertilized egg collected may be stored under freezing by the slow method or the ultrarapid method and the like.

Meanwhile, in the case of in vitro fertilization, a follicle-stimulating hormone and a luteinizing hormone are administered to a female non-human mammal for egg collection (the same as with in vivo fertilization is preferably used) as described above to induce ovulation, after which ova are collected and cultured in a medium for fertilization (e.g., TYH medium) in 5% gaseous carbon dioxide/95% atmosphere by the microdrop culture method and the like until in vitro fertilization. Separately, the cauda epididymidis is removed from a male non-human mammal of the same species (the same as with in vivo fertilization is preferably used), and a spermatozoa mass is collected and precultured in a medium for fertilization. After completion of the preculture, spermatozoa are added to the medium for fertilization containing the ova, and the ova are cultured in 5% gaseous carbon dioxide/95% atmosphere by the microdrop culture method and the like, after which a fertilized egg having two pronuclei is selected under a microscope. If DNA microinjection is not immediately performed, the fertilized egg obtained may be stored under freezing by the slow method or the ultrarapid method and the like.

DNA microinjection into the fertilized egg can be performed by a conventional method using a commonly known device such as a micromanipulator. Briefly, the fertilized egg placed in a microdrop of a medium for embryo culture is aspirated and immobilized using a holding pipette, and a DNA solution is injected directly into the male or female pronucleus, preferably into the male pronucleus, using an injection pipette. The introduced DNA is used preferably after being highly purified using CsCl density gradient ultracentrifugation or an anion exchange resin column and the like. It is also preferable that the introduced DNA be linearized in advance by cutting the vector portion using a restriction endonuclease.

After introducing the DNA, the fertilized egg is cultured in a medium for embryo culture in 5% gaseous carbon dioxide/95% atmosphere by the microdrop culture method and the like until the 1-cell stage to blastocyst stage, after which it is transplanted to the oviduct or uterus of a female non-human mammal for embryo reception rendered to be pseudopregnant. The female non-human mammal for embryo reception may be any one of the same species as the animal from which the early embryo to be transplanted is derived; for example, when a mouse early embryo is transplanted, a female ICR mouse (preferably about 8 to about 10 weeks of age) and the like are preferably used. A known method of rendering a female non-human mammal for embryo reception pseudopregnant is, for example, a method comprising mating the female with a vasectomized (vasoligated) male non-human mammal of the same species (for example, in the case of a mouse, with a male ICR mouse (preferably about 2 months or more of age)), and selecting a female confirmed to have a vaginal plug.

The female for embryo reception used may be one that has ovulated spontaneously, or one receiving luteinizing hormone releasing hormone (generally abbreviated as LHRH) or an analogue thereof administered prior to mating with a vasectomized (vasoligated) male, to induce fertility. Examples of the LHRH analogue include [3,5-DiI-Tyr⁵]-LH-RH, [Gln⁸]-LH-RH, [D-Ala⁶]-LH-RH, [des-Gly¹⁰]-LH-RH, [D-His(Bzl)⁶]-LH-RH and Ethylamides thereof and the like. The amount of LHRH or an analogue thereof administered, and the time of mating with a male non-human mammal after the administration vary depending on the species of the non-human mammal. For example, when the non-human mammal is a mouse (preferably an ICR mouse and the like), it is usually preferable that the female mouse be mated with a male mouse about 4 days after administration of LHRH or an analogue thereof; the amount of LHRH or an analogue thereof administered is usually about 10 to 60 µg/individual, preferably about 40 µg/individual.

Usually, if the early embryo to be transplanted is in the morula stage or after, the embryo is transplanted to the uterus of a female for embryo reception; if the early embryo is in a stage before the morula stage (for example, 1-cell stage to 8-cell stage embryo), the embryo is transplanted to the oviduct. The female for embryo reception is used as appropriate after elapse of a given number of days after becoming pseudopregnant depending on the developmental stage of the embryo to be transplanted. For example, in the case of a mouse, a female mouse at about 0.5 days after becoming pseudopregnant is preferable for the transplantation of a 2-cell stage embryo, and a female mouse at about 2.5 days after becoming pseudopregnant is preferable for the transplantation of a blastocystic embryo. After the female for embryo reception is anesthetized (preferably, Avertin, Nembutal and the like are used), an incision is made, the ovary is pulled out, and early embryos (about 5 to about 10 embryos) in suspension in a medium for embryo culture are injected into the vicinity of the abdominal osteum of the uterine tube or the uterine tube junction of the uterine horn using a pipette for embryo transplantation.

If the transplanted embryo implants successfully and the embryo recipient female becomes pregnant, non-human mammal pups will be obtained by spontaneous delivery or caesarian section. Embryo recipient females that have delivered spontaneously are allowed to continue suckling; if the pups are delivered by caesarian section, the pups can be suckled by a separately provided female for suckling (for example, in the case of the mouse, a female mouse with usual mating and delivery (preferably a female ICR mouse and the like)).

Transfer of the DNA that encodes an antisense RNA, siRNA, shRNA, or miRNA of SLC-1 in the fertilized egg cell stage is secured so that the introduced DNA will be present in all of the germline cells and somatic cells of the subject non-human mammal. Whether or not the introduced DNA is integrated in chromosome DNA can be determined by, for example, screening chromosome DNAs separated and extracted from the tail of the pup, by Southern hybridization or PCR. The presence of the targeting vector in the germline cells of the offspring non-human mammal (F₀) obtained as described above means that the targeting vector is present in all of the germline cells and somatic cells of all animals in the subsequent generation (F₁).
Usually, F₀ animals are obtained as heterozygotes having the introduced DNA in either of the homologous chromosomes. Different F₀ individuals have the introduced DNA inserted randomly on different chromosomes unless the insertion is by homologous recombination. To obtain a homozygote having the targeting vector in both of the homologous chromosomes, an F₀ animal and a non-transgenic animal are crossed to prepare an F₁ animal, and heterozygous siblings thereof having the introduced DNA in either of the homologous chromosomes may be crossed. If the introduced DNA is integrated only at one gene locus, 1/4 of the F₂ animals obtained will be homozygotes.

In another preferred embodiment with the use of a virus as the vector, as with the above-described case of KO animals, a method comprising infecting an early embryo or ES cell of a non-human mammal with a virus comprising a DNA that encodes an antisense RNA, siRNA, shRNA, or miRNA of SLC-1 can be mentioned. When a fertilized egg is used as the cell, it is preferable that the zone pallucida be removed prior to infection. After cultivation for 1 to 2 days following infection with the virus vector, the fertilized egg is transplanted to the oviduct or uterus of a female non-human mammal for embryo reception rendered to be pseudopregnant as described above in the case of an early embryo, or the fertilized egg is continued to be cultured with the addition of a selection drug as described above in the case of an ES cell, and a cell incorporating the vector is selected.

Furthermore, as described in the Proceedings of the National Academy of Sciences, USA (Proc. Natl. Acad. Sci. USA), vol.98, pp. 13090-13095, 2001, a spermatogonium collected from a male non-human mammal is infected with a virus vector during co-cultivation with STO feeder cells, after which the spermatogonium is injected into the seminiferous tube of a male infertile non-human mammal, and the male infertile non-human mammal is mated with a female non-human mammal, whereby pups that are hetero-Tg (+/-) for a DNA that encodes an antisense RNA, siRNA, shRNA, or miRNA of SLC-1 can be obtained efficiently.

The non-human mammal deficient in the expression of the SLC-1 gene of the present invention has the following characteristics:
(1) a lower blood insulin level in glucose tolerance test,
(2) increased insulin sensitivity,
(3) higher resistance to obesity even on high fat diet,
(4) a smaller white fat cell size, and
(5) accentuated lipolysis compared with the corresponding wild-type animal. These phenotypes have not been reported at least in conventionally publicly known SLC-1 KO mice (see Proceedings of the National Academy of Sciences, USA (Proc. Natl. Acad. Sci. USA), 2002, vol. 99, p. 3240-3245 and Endocrinology (Endocrinol.), 2002, vol. 143, p. 2469-2477).

Furthermore, the non-human mammal deficient in the expression of the SLC-1 gene of the present invention has the following characteristics:
(i) accentuated spontaneous movement and oxygen consumption,
(ii) decreased body fat, and
(iii) a decreased plasma leptin level compared with the corresponding wild-type animal. These phenotypes agree with those of conventionally publicly known SLC-1 KO mice.

On the basis of these findings, it is strongly suggested that SLC-1 may be not only involved in the onset and progression of obesity and diabetes through promoting food intake, but also involved profoundly in the exacerbation of glucose tolerance/insulin sensitivity. Therefore, the expression-deficient animal of the present invention can be utilized for, for example, the elucidation of the physiological functions of SLC-1 and the testing the efficacy of SLC-1 antagonist as a prophylactic/therapeutic drug for these diseases and the like, including the determination of the effect of SLC-1 deficiency on the pathologies of the diseases and the like, by being mated with various disease model animals (particularly obesity and/or type II diabetes model animals, or model animals for arteriosclerotic disease based commonly thereon) to render the disease model animals deficient in SLC-1.

A portion of the living body of an expression-deficient animal prepared as described above (for example, (1) a cell, tissue, organ and the like that are deficient in the expression of the SLC-1 gene, and (2) a cell or tissue derived therefrom, in culture, passaged as required, and the like) can also be used for the same purpose as that of the expression-deficient animal of the present invention. Examples of preferable portions of the living body of the expression-deficient animal of the present invention include organs such as the pancreas, liver, fat tissue, skeletal muscle, kidney, adrenal, blood vessel, heart, gastrointestinal tract, and brain, tissue sections and cells and the like derived from the organs.

In addition to having the expression of an endogenous SLC-1 gene inactivated, the SLC-1 non-human mammal deficient in the expression of the gene in the present invention may have one or more other gene modifications that produce the same or similar condition as a disease in which SLC-1 activity regulation is involved.
"A disease in which SLC-1 activity regulation is involved" is to be understood as a concept encompassing not only diseases resulting from an abnormality in SLC-1 activity or resulting in an abnormality in SLC-1 activity, but also diseases on which a prophylactic and/or therapeutic effect can be obtained by regulating SLC-1 activity.
For example, diseases that can be prevented/treated by inhibiting SLC-1 activity include obesity, hyperlipemia, type II diabetes and complications thereof (e.g., diabetic neuropathy, diabetic nephropathy, diabetic retinitis etc.), insulinoma, metabolic syndrome (including pathologic conditions wherein one or more of the aforementioned various diseases are concurrently present), arteriosclerotic disease (for example, myocardial infarction, angina pectoris, cerebral infarction, cerebral hemorrhage, cerebral thrombosis, cerebral embolism, aortic aneurysms, aortic dissociation, nephrosclerosis, renal insufficiency, obstructive arteriosclerosis, post-PCI restenosis, acute coronary syndrome, coronary arterial disease, peripheral arterial obstruction and the like), neuroses (for example, depression, anxiety and the like) and the like.

"Other gene modifications" include spontaneous disease model animals having an abnormality in an endogenous gene thereof due to a spontaneous mutation, Tg animals further incorporating another gene, KO/KD animals having an endogenous gene other than the SLC-1 gene inactivated (including Tg animals wherein gene expression has been reduced to an undetectable or negligible level by a gene destruction due to insertion mutation and the like, as well as introduction of an antisense DNA or a DNA that encodes a neutralizing antibody), dominant negative mutant Tg animals incorporating a mutant endogenous gene, and the like.

Examples of known "disease models having one or more other gene modifications that produce the same or similar condition as a disease in which SLC-1 activity regulation is involved" include NOD mice (Makino S. et al., Exp. Anim., vol. 29, page 1, 1980), BB rats (Crisa L. et al., Diabetes Metab. Rev.), vol. 8, page 4, 1992), ob/ob mouse, db/db mouse (Hummel L. et al., Science, vol. 153, page 1127, 1966), KK mouse, KKA^{y} mouse, GK rat (Goto Y. et al., Tohoku J. Exp. Med., vol. 119, page 85, 1976), Zucker fatty rat (Zucker L.M. et al., Ann. NY Acad. Sci., vol. 131, page 447, 1965), ZDF rat, OLETF rat (Kawano K. et al., Diabetes, vol. 41, page 1422, 1992) and the like as diabetes mellitus models, ob/ob mice, db/db mice, KK mouse, KKA^{y} mouse, Zucker fatty rat, ZDF rat, OLETF rat and the like as obesity models, WHHL rabbits (having mutation in low density lipoprotein receptor (LDLR); Watanabe Y., Atherosclerosis, vol. 36, page 261, 1980), SHLM (spontaneous mice having apoE deficiency mutation; Matsushima Y. et al., Mamm. Genome, vol. 10, page 352, 1999), LDLR KO mouse (Ishibashi S. et al., J. Clin. Invest., vol. 92, page 883, 1993), apoE KO mouse (Piedrahita J.A. et al., Proc. Natl. Acad. Sci. USA, vol. 89, page 4471, 1992), human apo A/human apoB double Tg mouse (Callow M.J. et al., Proc. Natl. Acad. Sci. USA, vol. 91, page 2130, 1994) and the like as hyperlipemia or arteriosclerosis models, ob/ob mice (Herberg L. and Coleman D.L., Metabolism, vol. 26, page 59, 1977), KK mouse (Nakamura M. and Yamada K., Diabetologia, vol. 3, page 212, page 1967), FLS mouse (Soga M. et al., Lab. Anim. Sci., vol. 49, page 269, 1999) as a fatty liver model, and CD55/CD59 double-Tg mice (Cowan P.J. et al., Xenotransplantation, vol. 5, pages 184-90, 1998) and the like as ischemic heart disease models.
These "disease models having other gene modifications" are purchasable from, for example, the Jackson Laboratory of the United States and the like, or can easily be prepared using a well known gene modification technology.

There is no particular limitation on the method of introducing one or more other gene modifications that will produce the same or similar pathologic condition as a disease involved by regulation of the activity of SLC-1 into the expression-deficient animal of the present invention; examples include (1) a method comprising crossing the expression-deficient animal of the present invention and a non-human mammal of the same species having one or more other gene modifications that will produce the same or similar condition as a disease involved by regulation of the activity of SLC-1; (2) a method comprising treating an early embryo or ES cell of a non-human mammal having one or more other gene modifications that produce the same or similar condition as a disease involved by regulation of the activity of SLC-1, by the above-described method, to inactivate the expression of an endogenous SLC-1 gene to obtain a KO/KD animal; (3) a method comprising introducing one or more other gene modifications that will produce the same or similar condition as a disease involved by regulation of the activity of SLC-1 into an early embryo or ES cell of a non-human mammal having an endogenous SLC-1 gene inactivated by the method described above, and the like. If one or more other gene modifications that produce the same or similar condition as a disease in which SLC-1 activity regulation is involved are achieved by introducing an exogenous gene or dominant mutant gene, the exogenous gene and the like and a targeting vector/ a DNA that encodes antisense RNA or siRNA may be introduced simultaneously or sequentially into an early embryo or ES cell of a wild type non-human mammal to obtain a KO/KD animal.

If the expression deficient animal of the present invention is crossed with a disease model non-human mammal of the same species having one or more other gene modifications that produce the same or similar condition as a disease in which SLC-1 activity regulation is involved, it is desirable that homozygotes be crossed. For example, the F₁ obtained by crossing a homozygous mouse that is deficient in the expression of the SLC-1 gene and a KKA^{y} mouse (obesity/type II diabetes model) will be SLC-1(+/-)×KKA^{y} or SLC-1(+/-)×KK at a probability of 1/2. By crossing the F₁ individuals of SLC-1(+/-)×KKA^{y} and SLC-1(+/-)×KK, SLC-1(-/-)×KKA^{y} is obtained at a probability of 1/8. Acquirement of homo-individuals in F₃ and subsequent generations can be achieved by crossing SLC-1(-/-)×KKA^{y} and SLC-1(-/-)xKK (homo-individuals are acquired at a probability of 1/2).

The expression deficient animal of the present invention may have undergone one or more non-genetic treatments that produce the same or similar condition as a disease in which SLC-1 activity regulation is involved. "A non-genetic treatment" means a treatment that does not produce a gene modification in the subject non-human mammal. Examples of such treatments include, but are not limited to, induction with drugs such as STZ, dietary stress loads such as high-fat diet load, glucose load, and fasting, external stress loads such as UV, active oxygen, fever, and blood vessel ligation/reperfusion and the like.

Preferably, as "a disease model having one or more other gene modifications that will produce the same or similar condition as the condition of a disease involved by regulation of the activity of SLC-1", an obesity and/or type II diabetes model, particularly preferably a KKA^{y} mouse, can be mentioned. Accordingly, the present invention also provides an obesity and/or type II diabetes model non-human mammal that is deficient in the expression of the SLC-1 gene (preferably KKA^{y} mouse).
The obesity and/or type II diabetes model non-human mammal that is deficient in the expression of the SLC-1 gene of the present invention has the following characteristics:
(1) elevated adiponectin expression,
(2) delayed onset of hyperglycemia,
(3) a lower blood glycohemoglobin level, and
(4) accentuated energy consumption
compared with the corresponding obesity and/or type II diabetes model non-human mammal wherein the expression of the gene is normal. None of these phenotypes have been reported at least in conventionally publicly known SLC-1 deficient/obesity/type II diabetes model mice (see Diabetes, 2006, vol. 55, p. 725-733).

Furthermore, the obesity and/or type II diabetes model non-human mammal that is deficient in the expression of the SLC-1 gene of the present invention has the following characteristics:
(i) increased oxygen consumption, and
(ii) a decreased blood corticosterone level
compared with the corresponding obesity and/or type II diabetes model non-human mammal wherein the expression of the SLC-1 gene is normal. These phenotypes agree with those of conventionally publicly known SLC-1-deficient/obesity/type II diabetes model mice.

As stated above, in the obesity and/or type II diabetes model non-human mammal that is deficient in the expression of the SLC-1 gene of the present invention, compared with the corresponding obesity and/or type II diabetes model non-human mammal wherein the expression of the gene is normal, adiponectin expression is increased. This strongly suggests that SLC-1 antagonists may have the action of promoting adiponectin production in fat cells in animal individuals with obesity, particularly with visceral fat type obesity accompanied by fat cell hypertrophy.
Accordingly, the present invention also provides a promoter of adiponectin production comprising an SLC-1 antagonist. Here, "an antagonist" is "a substance possessing antagonist activity", and "antagonist activity" refers to the property of antagonistically binding to the ligand binding site of SLC-1, but having almost no or totally no influence on the equilibrium between the active form and the inactive form, or the property of binding to an optionally chosen site of SLC-1 to shift the equilibrium between the active form and the inactive form of SLC-1 toward the more inactive side. Therefore, as mentioned herein, "an antagonist" is to be defined as a concept that encompasses both what is called neutral antagonists and inverse agonists.

The promoter of adiponectin production of the present invention, which comprises an SLC-1 antagonist, can be used for, for example, the prevention and/or treatment of hyperlipemia, type II diabetes and complications thereof (for example, diabetic neuropathy, diabetic nephropathy, diabetic retinopathy and the like), insulin resistance syndrome, hypertension, cancers, including insulinoma, metabolic syndrome (including pathologic conditions wherein one or more of the aforementioned various diseases are concurrently present), arteriosclerotic diseases (for example, myocardial infarction, angina pectoris, cerebral infarction, cerebral hemorrhage, cerebral thrombosis, cerebral embolism, aortic aneurysms, aortic dissociation, nephrosclerosis, renal insufficiency, obstructive arteriosclerosis, post-PCI restenosis, acute coronary syndrome, coronary arterial disease, peripheral arterial obstruction and the like) and the like in mammals with obesity.

Examples of SLC-1 antagonists include, but are not limited to, the compounds described in WO 01/21577, WO 01/82925, WO 01/87834, WO 03/35624, WO 2004/072018 and elsewhere, and the like. For example, SLC-1 antagonists selected by the screening methods described in WO 00/40725 and the like can also be used preferably.

SLC-1 antagonists can, for example, be used orally as tablets coated with sugar as required, capsules, elixirs, microcapsules and the like, or can be used parenterally in the form of an injection such as a sterile solution or suspension in water or another pharmaceutically acceptable liquid. The antagonists can be prepared as pharmaceutical preparations by being blended with a physiologically acceptable carrier, flavoring agent, filler, vehicle, antiseptic, stabilizer, binder and the like, in a unit dosage form required for generally accepted preparation design. The amounts of active ingredients in these preparations are chosen as appropriate in consideration of the doses described below.

Examples of additives that can be blended in tablets, capsules and the like include binders like gelatin, cornstarch, tragacanth and acacia, fillers like crystalline cellulose, swelling agents like cornstarch, gelatin, alginic acid and the like, lubricants like magnesium stearate, sweeteners like sucrose, lactose or saccharin, flavoring agents like peppermint, acamono oil or cherry, and the like. When the formulation unit form is a capsule, the above-described type of material can further contain a liquid carrier like an oil or fat. A sterile composition for injection can be formulated according to an ordinary procedure for pharmaceutical making, such as dissolving or suspending an active substance in a vehicle like water for injection, or a naturally occurring vegetable oil such as sesame oil or coconut oil.

The aqueous solution for injection is exemplified by saline, isotonic solutions containing glucose and another auxiliary (for example, D-sorbitol, D-mannitol, sodium chloride and the like) and the like, and may be used in combination with an appropriate solubilizer, for example, an alcohol (e.g., ethanol), a polyalcohol (for example, propylene glycol, polyethylene glycol and the like), a nonionic surfactant (for example, Polysorbate 80^{™}, HCO-50 and the like) and the like. The oily liquid is exemplified by sesame oil, soybean oil and the like, and may be used in combination with a solubilizer such as benzyl benzoate or benzyl alcohol. Also, the aqueous solution for injection may be formulated with, for example, a buffering agent (for example, phosphate buffer solution, sodium acetate buffer solution and the like), a soothing agent (for example, benzalkonium chloride, procaine hydrochloride and the like), a stabilizer (for example, human serum albumin, polyethylene glycol and the like), a preservative (for example, benzyl alcohol, phenol and the like), an antioxidant and the like. The injectable preparation prepared is usually filled in an appropriate ampoule.

Because the preparation thus obtained is safe and of low toxicity, it can be administered to, for example, mammals (for example, humans, rats, mice, guinea pigs, rabbits, sheep, pigs, bovines, horses, cats, dogs, monkeys and the like). As described above, SLC-1 deficiency is effective in elevating the adiponectin level when obesity is present. That is, in animal individuals with obesity, particularly with visceral fat type obesity accompanied by fat cell hypertrophy, adiponectin production/secretion in fat cells is suppressed; if SLC-1 activity is inhibited, the adiponectin level in plasma shows a tendency for recovery. On the other hand, in animal individuals without obesity, there is essentially no decrease in adiponectin production/secretion, and the adiponectin level does not rise even if SLC-1 is deleted. Therefore, the promoter of adiponectin production of the present invention, wherein an SLC-1 antagonist is an active ingredient, is preferably administered to the above-described mammal having the adiponectin level decreased because of obesity, particularly visceral fat type obesity accompanied by fat cell hypertrophy and the like.

The dose of the SLC-1 antagonistic drug varies depending on the target disease, subject of administration, route of administration and the like; for example, in the case of oral administration for treatment of diabetes mellitus accompanying lower level of adiponectin, the usual dosage for an adult (weighing 60 kg) is about 0.1 mg to about 100 mg, preferably about 1.0 to about 50 mg, more preferably about 1.0 to about 20 mg, per day. In the case of parenteral administration, the dose of the antagonistic drug varies depending on the subject of administration, target disease and the like; for example, in the case of administration as an injection to an adult (weighing 60 kg) for treatment of diabetes mellitus accompanying lower level of adiponectin, the dose is about 0.01 to about 30 mg, preferably about 0.1 to about 20 mg, more preferably about 0.1 to about 10 mg, per day. If the subject of administration is a non-human animal, an amount converted per 60 kg of body weight can be administered.

In the description of the present application, the codes of bases, amino acids and the like are denoted in accordance with the IUPAC-IUB Commission on Biochemical Nomenclature or by the common codes in the art. Examples thereof are as follows.
- DNA: :deoxyribonucleic acid
- cDNA: :complementary deoxyribonucleic acid
- A: :adenine
- T: :thymine
- G: :guanine
- C: :cytosine
- RNA: :ribonucleic acid
- mRNA: :messenger ribonucleic acid
- dATP: :deoxyadenosine triphosphate
- dTTP: :deoxythymidine triphosphate
- dGTP: :deoxyguanosine triphosphate
- dCTP: :deoxycytidine triphosphate
- ATP: :adenosine triphosphate
- EDTA: :ethylenediaminetetraacetic acid
- SDS: :dodecylsodium sulfate
- Gly: :glycine
- Ala: :alanine
- Val: :valine
- Leu: :leucine
- Ile: :isoleucine
- Ser: :serine
- Thr: :threonine
- Cys: :cysteine
- Met: :methionine
- Glu: :glutamic acid
- Asp: :aspartic acid
- Lys: :lysine
- Arg: :arginine
- His: :histidine
- Phe: :phenylalanine
- Tyr: :tyrosine
- Trp: :tryptophan
- Pro: :proline
- Asn: :asparagine
- Gln: :glutamine
- pGlu: :pyroglutamic acid
- Me: :methyl croup
- Et: :ethyl group
- Bu: :butyl group
- Ph: :phenyl group
- TC: :thiazolidine-4(R)-carboxamide group

### [Examples]

The present invention is hereinafter described in detail by means of the following examples, to which, however, the invention is never limited.

### Example 1 Preparation of mice deficient in the SLC-1 gene

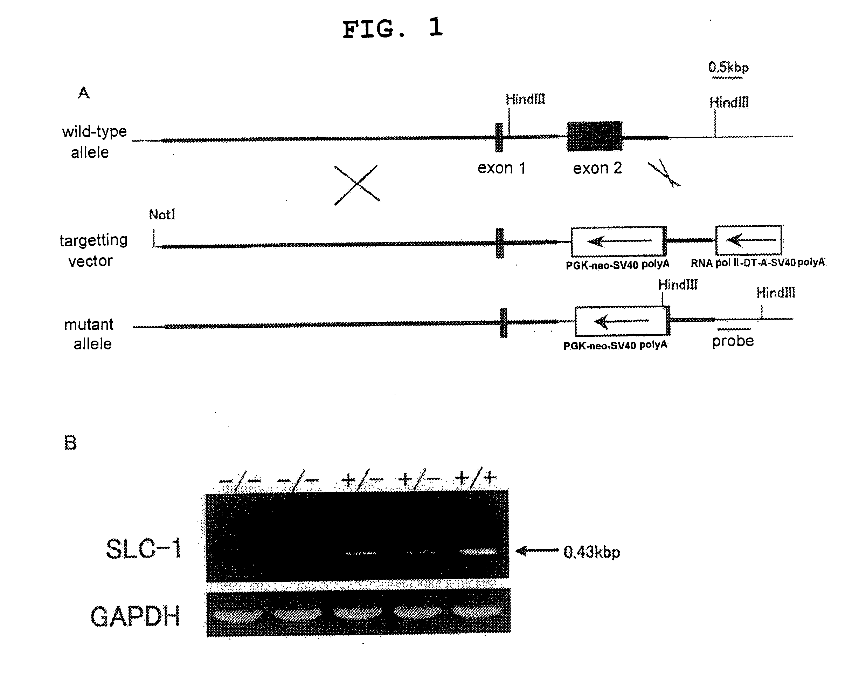

The plasmid pSLCTA-2 comprising a targeting vector (FIG. 1A) was prepared by cloning with a 7.7-kbp XbaI fragment comprising the exon 1 of mouse SLC-1 genomic DNA and a 0.87-kbp portion of the exon 2 from SacI to the EcoRI of the 3' nontranslated region as the 5' arm and 3' arm, respectively, then introducing them into pKOScrambler (produced by Lexicon Genetics), and replacing the 7 transmembrane region of the exon 2 with the neomycin resistance gene. The targeting vector was linearized by NotI cleavage, and electroporated into 129SvEv mouse-derived ES cell AB2.2 (produced by Lexicon Genetics) using a gene pulser (produced by Bio-Rad), after which the cells were subjected to selection culture with the neomycin analogue G418 (produced by Lexicon Genetics). From 480 cells of a G418 resistant line, genomic DNA was extracted; PCR screening was performed using the NE5 primer (5'-CTAAAGCGCATGCTCCAGAC-3': SEQ ID NO:1) in the neomycin resistance gene (neo) sequence and the MC18 primer (5'-ATATCAGGTATTAGAGTGAC-3': SEQ ID NO:2) of the sequence outside of the 3' arm, and 14 homologous recombinant strains were selected. Furthermore, genomic DNA extracted from each homologous recombinant strain was cleaved with HindIII, Southern hybridization was performed using a probe outside the 3' arm, and a 3.5-kbp fragment from the wild type and a 1.5-kbp fragment from a homologous recombinant strain were identified. Each homologous recombinant strain was micro-injected into a blastocyst of a C57BL/6J mouse to acquire a germline male chimeric mouse. The germline male chimeric mouse and a female C57BL/6J mouse were mated to obtain offspring pups, and the genotypes thereof were determined by performing PCR with a DNA extracted from the tail as the template, using the NE1 primer in the neo gene sequence (5'-CCGCTTCCATTGCTCAGCGG-3': SEQ ID NO:3), the MC19 primer in the deleted exon 2 region (5'-GCTTGGTGCTGTCGGTGAAG-3': SEQ ID NO:4) and the MC14 primer in the 3' arm (5'-TATTCTGTCAAGGGGATC-3': SEQ ID NO:5). The presence or absence of the expression of the SLC-1 gene in the pups was determined by performing reverse transcription-PCR with the reverse transcription product of total RNA extracted from the whole brain using ISOGEN (produced by Nippon Gene) as the template, using the MC26 primer (5'-CCTCGCACAAGGAGTGTCTC-3': SEQ ID NO:6) and MC29 primer (5'-TAATGAACGAGAGAGCCCAC-3': SEQ ID NO:7) placed in the deleted exon 2 region, on the basis of the amplifiability of the mRNA-derived 0.43-kbp band was identified (FIG. 1B).
From the individual resulting from crossing of the ES cell-derived 129SvEv strain and the C57BL/6J strain used for mating, a non-congenic strain was prepared. Separately, the non-congenic strain was back-crossed to a C57BL/6J mouse for 4 generations by the speed congenic method, after which a congenic strain was prepared by sibling mating.

### Example 2 Preparation of individuals resulting from hybridization of KKA^{y} mice or KK mice and SLC-1 homo-deficient (-/-) mice

By crossing an SLC-1(-/-) mouse made to be congenic and a KKA^{y} mouse, SLC-1 hetero-deficient (+/-) mice incorporating 50% of the genetic background of the KKA^{y} mouse or KK mouse were acquired. By intercrossing each, SLC-1 wild (+/+) mouse strains [KKA^{y}/SLC-1(+/+), KK/SLC-1(+/+)] and SLC-1(-/-) mouse strains [KKA^{y}/SLC-1(-/-), KK/SLC-1(-/-)] were acquired.

### Example 3 General properties of SLC-1(-/-) mice

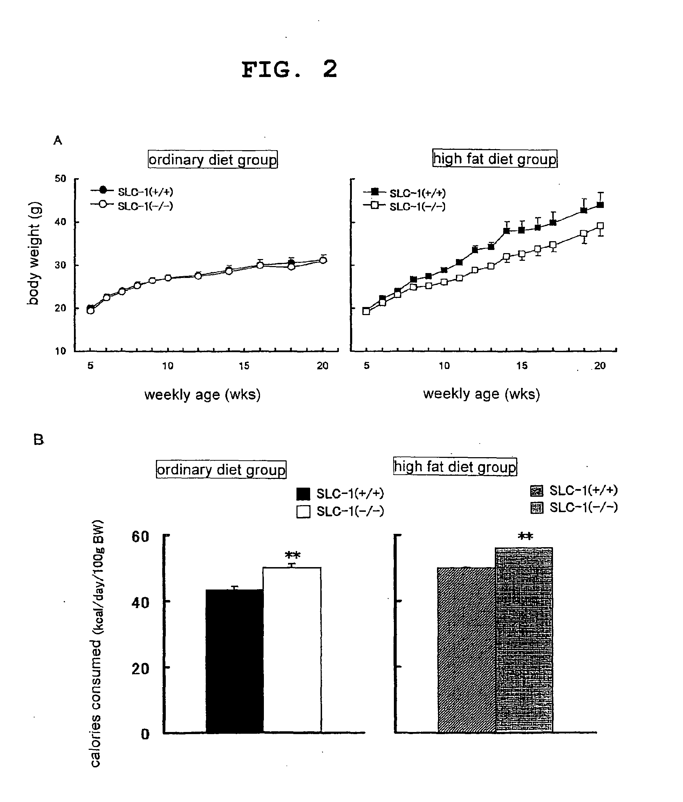

SLC-1(+/+) mice and SLC-1(-/-) mice were individually reared under the conditions of a 12-hour lighting cycle at a room temperature of 24±1°C and a humidity of 55±5% from 5 weeks of age. The feed used was an ordinary diet (CE-2, 11.6% kcal from fat, 346.8 kcal/100 g, produced by Clea Japan), or a high fat diet containing unsalted butter (40.7% kcal from fat, 464.6 kcal/100 g, produced by Clea Japan). Body weight was measured from 8:00 am on the specified days of each week. For food intake, weekly food intake was measured, and converted to daily calorific intake per 100 g of body weight using the calculation formula of weekly food intake (g) x calorific value of food (kcal/100 g)/body weight (g)/7 (day). For blood parameters, orbital blood was drawn under satiation from 8:00 am using a heparinized blood drawing tube (produced by Drummond Scientific Company) at 12 weeks of age and 21 weeks of age, and glucose (DRI-CHEM System, produced by Fuji Photo Film), triglycerides (DRI-CHEM System, produced by Fuji Photo Film), total cholesterol (DRI-CHEM System, produced by Fuji Photo Film), insulin (Levis Insulin Kit, produced by Shibayagi), leptin (produced by mouse leptin kit, Genzyme-Techne), and nonesterified fatty acids (NEFA, NEFA C-Test Wako, produced by Wako Pure Chemical Industries) in plasma were measured.
Regarding body weight, the SLC-1(-/-) mice, compared with the SLC-1(+/+) mice, in the ordinary diet group, throughout the experimental period (at 5 to 20 weeks of age), did not exhibit a significant difference, but in the high fat diet group, the body weight was smaller beyond 6 weeks of age (P<0.01, at 8 weeks of age) (FIG. 2A). For daily food intake (kcal/day), an increase was observed in the SLC-1(-/-) mice in the ordinary diet group, but no difference was observed between the two types of mice in the high fat diet group (data not published). Food intake as corrected for body weight was higher in the SLC-1(-/-) mice in both the ordinary diet and high fat diet groups (FIG. 2B). Regarding plasma parameters, in the ordinary diet group, glucose, triglycerides, total cholesterol, and free fatty acids showed no difference between the SLC-1(+/+) mice and the SLC-1(-/-) mice, but the leptin and insulin levels were significantly lower in the SLC-1(-/-) mice. In the high fat diet group, the same tendency as the ordinary diet group was observed (Table 1).

**Table 1**

| Plasma parameters in SLC-1 deficient mice | | | | |
|---|---|---|---|---|
| | Ordinary diet group | | High fat diet group | |
| | SLC-1(+/+) | SLC-1(-/-) | SLC-1(+/+) | SLC-1(-/-) |
| Glucose (mg/dl) | 185±9 | 187±10 | 219±6 | 204±9 |
| Triglycerides (mg/dl) | 99±4 | 85±8 | 103±6 | 89±4 |
| Total cholesterol (mg/dl) | 114±11 | 92±8 | 218±8 | 190±8* |
| Leptin (ng/ml) | 3.9±0.6 | 1.4±0.2** | 9.4±1.4 | 3.9±0.8** |
| Free fatty acids (mEq/l) | 0.20±0.03 | 0.18±0.03 | 0.39±0.03 | 0.41±0.04 |
| Insulin (ng/ml) | 1.4±0.2 | 0.7±0.1 | 7.1±2.2 | 2.3±0.6 |

| | | | | |
|---|---|---|---|---|
| Male mice were used. Glucose, triglycerides, total cholesterol, leptin, and free fatty acids were measured at 12 weeks of age, and insulin at 21 weeks of age. n=10, Mean±S.E., *, P<0.05; **, P<0.01 vs. each SLC-1(+/+). | | | | |

### Example 4 Adipose tissue of SLC-1(-/-) mice

Body fat percentages, adipose tissue weights, and white fat cell sizes were measured at 12 to 14 weeks of age. The body fat percentages were measured under Nembutal anesthesia by double-energy X-ray absorptiometry (DEXA, QDR-4500a Rat Whole Body V8.26a, produced by HOLOGIC). The adipose tissue weight was measured on retroperitoneal, perigenital, mesenteric, perirenal, and subcutaneous inguinal portions for white adipose tissue, and on the interscapulum for brown adipose tissue. White fat cell size was measured as described below. Fat cells were prepared in accordance with the method of Rodbell (Rodbell, M. (1964) Metabolism of isolated fat cells. I. Effects of hormones on glucose metabolism and lipolysis. J. Biol. Chem. 239:375-380). After extirpation, mouse perigenital white adipose tissue was shredded on parchment paper, and added into a Krebs-Ringer bicarbonate buffer solution containing 3% BSA (Albumin, bovine serum, fraction V, fatty acid-free, produced by Wako Pure Chemical Industries) and 0.075% collagenase type I (produced by Worthington Biochemical), after which a 95% O₂ - 5% CO₂ gas was blown into the gas phase, and the liquid was shaken at 37°C, 120 strokes/minute for 35 minutes. The suspended fat cells were filtered through meshed cloth, and tissue fragments were removed. After the filtrate was allowed to stand, the liquid layer was removed, and the fat cell layer was washed with a Krebs-Ringer bicarbonate buffer solution containing 1% BSA (15 to 20 ml) 3 times. After suspending, the fat cell suspension was mounted on a siliconized glass slide, and photographed at a magnifying rate of 200 fold under an inverted microscope, and the diameters of the cells were measured (cell count ≥ 180).
The SLC-1(-/-) mice, both in the ordinary diet group and the high fat diet group, exhibited significantly lower values of body fat percentage as determined by the DEXA method (Table 2). Regarding adipose tissue weights, a decreasing tendency or a significant decrease was observed in retroperitoneal adipose tissue, perigenital adipose tissue, mesenteric white adipose tissue, and interscapular brown adipose tissue, in the ordinary diet group, but this difference became more conspicuous under conditions for induction of obesity by high fat diet loading, and the weight of adipose tissue decreased significantly at all measurement sites including perirenal and subcutaneous adipose tissue (Table 2). The fat cell size for perigenital white adipose tissue was significantly smaller for the SLC-1(-/-) mice, compared with the SLC-1(+/+) mice, in both groups loaded with the ordinary diet or the high fat diet, respectively (Table 2).

**Table 2**

| Changes in body fat percentage, adipose tissue weight, and fat cell size for SLC-1 deficient mice | | | | |
|---|---|---|---|---|
| | Ordinary diet group | | High fat diet group | |
| | SLC-1(+/+) | SLC-1(-/-) | SLC-1(+/+) | SLC-1(-/-) |
| Body fat percentage (% BW) | 10.2±0.9 | 6.9±0.5** | 31.8±3.2 | 15.6±2.0** |
| Retroperitoneal white adipose tissue (mg) | 52.8±7.5 | 35.5±3.8 | 312.4±38.7 | 136.5±31.0** |
| Perigenital white adipose tissue (mg) | 253.2±16.4 | 207.4±15.8 | 1212.0±145.3 | 494.7±93.9** |
| Mesenteric white adipose tissue (mg) | 275.0±23.2 | 225.6±13.8 | 580.1±70.6 | 291.3±49.7** |
| Perirenal white adipose tissue (mg) | 29.9±3.0 | 27.8±1.9 | 114.9±21.0 | 62.4±10.6* |
| Subcutaneous white adipose tissue (mg) | 147.1±16.4 | 158.9±10.5 | 821.3±104.0 | 360.3±46.2** |
| Interscapular brown adipose tissue (mg) | 126.9±13.0 | 71.5±4.1** | 183.4±12.0 | 120.0±8.7** |
| Perigenital white fat cell diameter (µm) | 68.4±0.9 | 52.8±0.6** | 93.4±1.7 | 70.8±0.8** |

| | | | | |
|---|---|---|---|---|
| The measurements were taken using male mice at 12 to 14 weeks of age. Body fat percentage, adipose tissue weight data: n=10, mean±S.E., fat cell size: cell count ≥ 180, mean±S.E., *, P<0.05; **, P<0.01 vs. each SLC-1(+/+). | | | | |

### Example 5 Energy consumption by SLC-1(-/-) mice

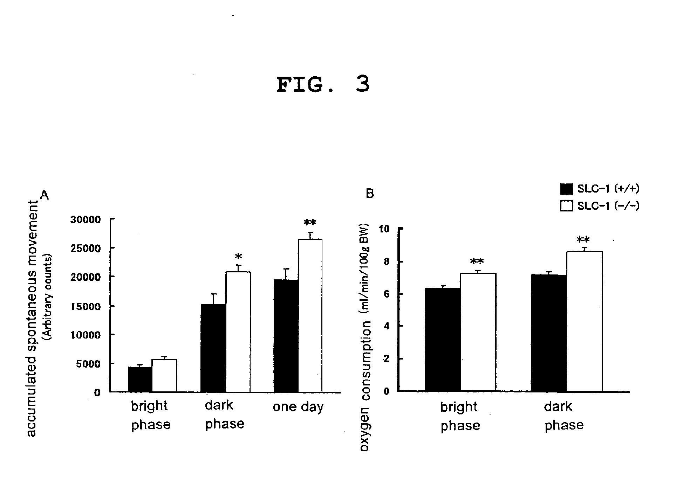

Spontaneous movement was measured at 12 weeks of age using an infrared behavior analyzer (ABsystem3.04, NeuroScience). The measurements were taken for 2 days after acclimation under satiation. Movement changes of 0.5 seconds or more were counted, and the results were shown as mean values for the counts in the bright phase or dark phase of 2 days. Oxygen consumption was measured using a small animal metabolism measuring system (model MK-5000RQ/06, Muromachi Kikai). Each mouse was placed in an airtight chamber [150W x 150D x 150H (mm)], and the measurements were taken at an air flow rate of 0.5-0.8 L/min for 24 hours. Free access to food and water was allowed. The air was sampled both inside and outside the chamber every 5 minutes 15 seconds, and the oxygen concentration (%) or carbon dioxide concentration (%) in each sample was measured. Oxygen consumption (VO₂: ml/min/100 g BW) or carbon dioxide emission (VCO₂: ml/min/100 g BW) was calculated by multiplying the concentration difference between the inside and outside of the chamber by the air flow rate, and corrected to obtain a value per 100 g of mouse body weight. Respiratory quotient (RQ) was calculated using the calculation formula of carbon dioxide emission (VCO₂)/oxygen consumption (VO₂). The results were shown as mean values for the counts in the bright phase or dark phase.
In SLC-1(-/-) mice, spontaneous movement was accentuated by 37% in the dark phase, and daily cumulative movement was accentuated by 36% (FIG. 3A). Oxygen consumption increased significantly in both the bright phase and dark phase (FIG. 3B). In the respiratory quotient, which reflects the metabolic ratio of the three major nutrients (carbohydrates, proteins, and lipids), no difference was observed (data not published).

### Example 6 Glucose tolerance test and insulin tolerance test on SLC-1(-/-) mice

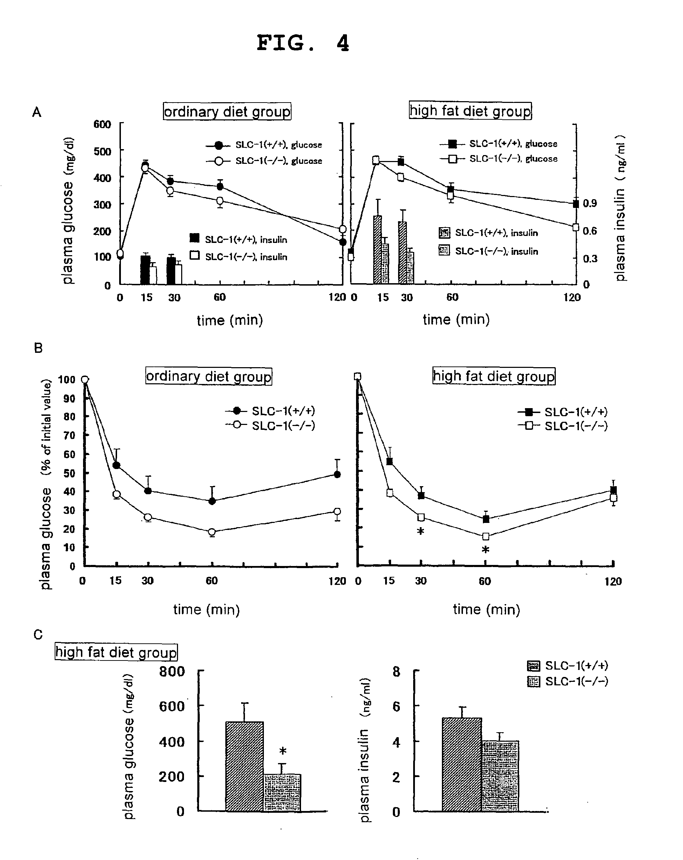

A glucose tolerance test was performed by the method described below. Specifically, mice reared under loading with an ordinary diet or a high fat diet were fasted for 20 hours, after which glucose (1 g/kg) was administered orally, and orbital blood was drawn using a heparinized blood drawing tube (produced by Drummond Scientific Company) after elapse of 0, 5, 15, 30, 60, and 120 minutes. The glucose level (produced by DRI-CHEM System, Fuji Photo Film) and insulin level (mouse insulin kit, produced by Shibayagi) in plasma were measured.
An insulin tolerance test was performed by the method described below. Specifically, mice reared under loading with an ordinary diet or a high fat diet were fasted for 20 hours, after which insulin (0.75 U/kg, produced by Novo Nordisk Pharma) was injected intraperitoneally. After elapse of 0, 15, 30, 60, and 120 minutes, orbital blood was drawn using a heparinized blood drawing tube (produced by Drummond Scientific Company), and the glucose level in plasma (DRI-CHEM System, produced by Fuji Photo Film) was measured.
An insulin resistance test (steady state plasma glucose: SSPG method) was performed by the method described below. Specifically, mice reared under loading with a high fat diet were fasted for 20 hours, after which a mixed liquid of insulin (1 U/kg, produced by Novo Nordisk Pharma), glucose (3 g/kg, produced by Wako Pure Chemical Industries), epinephrine (100 µg/kg, produced by Sigma-Aldrich), and propranolol (5 mg/kg, produced by Sigma-Aldrich) was administered subcutaneously. After 50 and 75 minutes, when the blood glucose level and insulin level became stationary, orbital blood was drawn, and the glucose level (DRI-CHEM System, produced by Fuji Photo Film) and insulin level (mouse insulin kit, produced by Shibayagi) in plasma were measured. The results are shown as mean values for 50 minutes and 75 minutes.
In the glucose tolerance test at 16 weeks of age, no difference in the plasma glucose levels before and after glucose loading was observed in the SLC-1(-/-) mice, compared with the SLC-1(+/+) mice, in both the ordinary diet group and the high fat diet group, but the plasma insulin level was kept low (FIG. 4A), and insulin sensitivity was higher in the SLC-1(-/-) mice.
In the insulin tolerance test at 21 weeks of age, the SLC-1(-/-) mice, compared with the SLC-1(+/+) mice, exhibited lower values of plasma glucose after administration of insulin (FIG. 4B). Furthermore, to clarify the changes during loading with a high fat diet, a test to evaluate insulin resistance in peripheral tissue (SSPG method) was performed at 29 weeks of age. In the SLC-1(-/-) mice, compared with the SLC-1(+/+) mice, the SSPG level was significantly lower under conditions that did not produce a difference in steady state plasma insulin (SSPI) level (FIG. 4C).

### Example 7 Lipolysis in SLC-1(-/-) mice

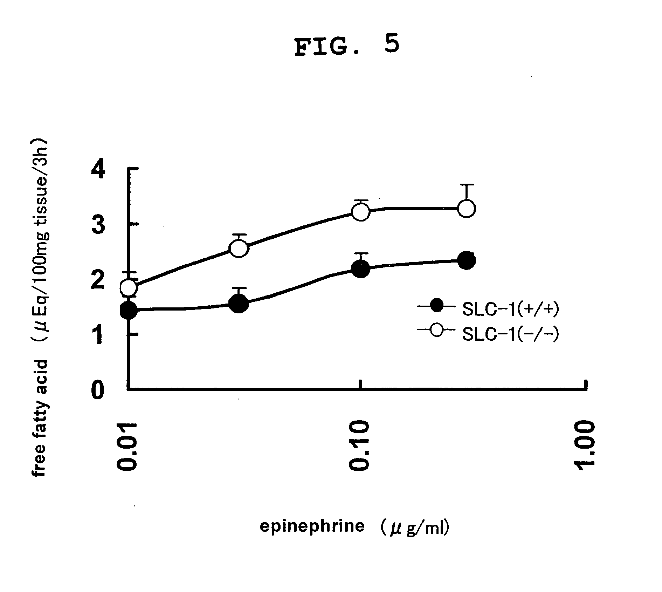

After extirpating bilaterally, perigenital white adipose tissue was washed with a Krebs-Ringer bicarbonate buffer solution containing 2% BSA (Albumin, bovine serum, fraction V, fatty acid-free, produced by Wako Pure Chemical Industries), and each was halved. Each tissue section was deprived of excess water, and then weighed and immersed in 1 ml of a Krebs-Ringer bicarbonate buffer solution containing epinephrine at various concentrations (0, 0.01, 0.03, 0.1, 0.3 µg/ml, produced by Sigma-Aldrich) and 2% BSA. A 95% O₂- 5% CO₂ gas was blown into the gas phase, and the liquid was shaken at 37°C, 80 strokes/minute for 3 hours. After shaking, the free fatty acids in the reaction liquid (NEFA C-Test Wako, produced by Wako Pure Chemical Industries) were measured.
Although the reactivity with the addition of epinephrine, which accentuates lipolysis, was accentuated in SLC-1(-/-) mice, compared with SLC-1(+/+) mice (P<0.01), no difference was observed between the two groups (FIG. 5).

### Example 8 General properties of KKA^{y} mouse and KK mouse hybrid groups

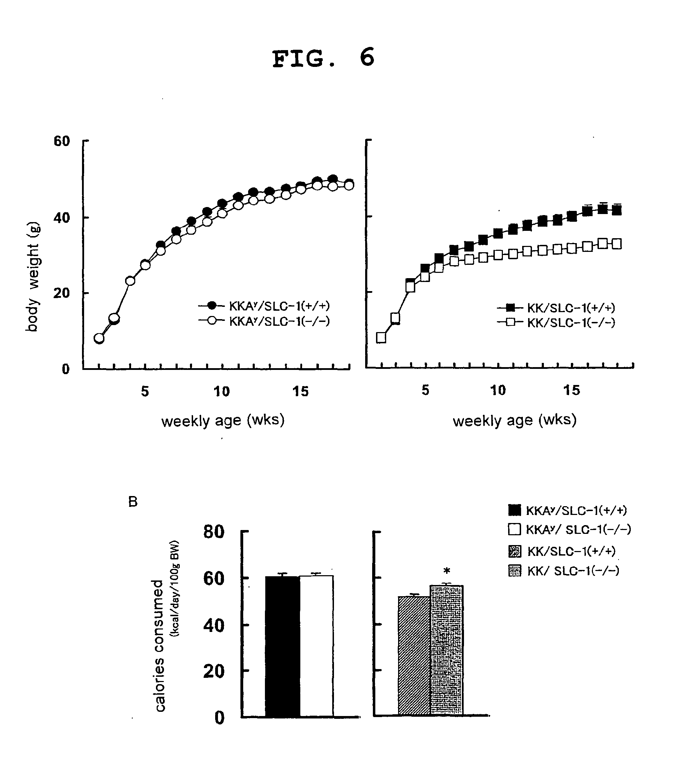

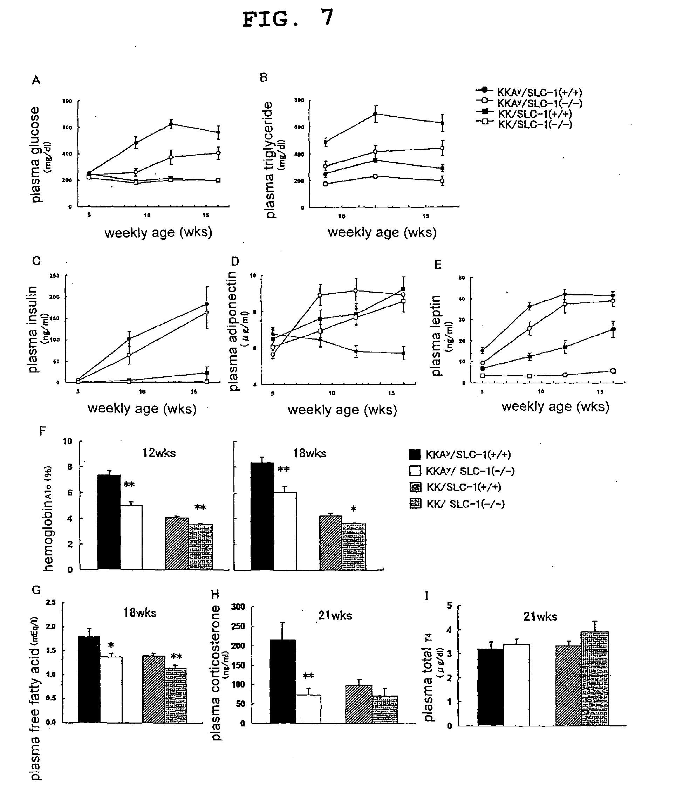

SLC-1 wild (+/+) mouse strains [KKA^{y}/SLC-1(+/+), KK/SLC-1(+/+)] and SLC-1(-/-) mouse strains [KKA^{y}/SLC-1(-/-), KK/SLC-1(-/-)] were individually reared under conditions of a 12-hour lighting cycle at a room temperature of 24±1°C and a humidity of 55±5%. The food given was an ordinary diet (CE-2, 11.6% kcal from fat, 346.8 kcal/100 g, produced by Clea Japan). Body weight was measured from 8:00 am on the specified day every week. Regarding food intake, weekly food intake was measured and converted to daily calorific intake per 100 g of body weight using the calculation formula of weekly food intake (g) x calorific value of food (kcal/100 g)/body weight (g)/7 (day). For blood parameters, orbital blood was drawn under satiation using a heparinized blood drawing tube (produced by Drummond Scientific Company) from 8:00 am, and glucose (DRI-CHEM System, produced by Fuji Photo Film), triglycerides (DRI-CHEM System, produced by Fuji Photo Film), total cholesterol (DRI-CHEM System, produced by Fuji Photo Film), insulin (Levis Insulin Kit, produced by Shibayagi), leptin (mouse leptin kit, produced by Genzyme-Techne), adiponectin (mouse adiponectin RIA kit, produced by Linco Research), and nonesterified fatty acids (NEFA, NEFA C-Test Wako, produced by Wako Pure Chemical Industries) in plasma and hemoglobin A1c (HbA1c: glycohemoglobin, automated glycohemoglobin analyzer HLC-723GHbV A1c2.2, produced by Tosoh Corporation) in whole blood were measured. After blood was drawn from the carotid artery under satiation, corticosterone (corticosterone ¹²⁵I RIA kit, produced by ICN Biomedicals) and total T4 (DPC/total T4, produced by Mitsubishi Kagaku Iatron) in plasma prepared by drawing blood from the carotid artery under satiation, and adding a 1/100 volume of 2% EDTA solution, were measured.
No difference in body weight was observed between the KKA^{y}/SLC-1(+/+) mice and the KKA^{y}/SLC-1(-/-) mice, but between the KK/SLC-1(+/+) mice and the KK/SLC-1(-/-) mice, beyond 6 weeks of age, the KK/SLC-1(-/-) mice exhibited significantly lower body weight gain (P<0.05, at 6 weeks of age) (FIG. 6A). In daily food intake by mice (kcal/day), no difference was observed between the KKA^{y}/SLC-1(+/+) mice and the KKA^{y}/SLC-1(-/-) mice, and between the KK/SLC-1(+/+) mice and the KK/SLC-1(-/-) mice (data not published). However, when daily food intake was corrected for body weight, no difference was observed between the KKA^{y}/SLC-1(+/+) mice and the KKA^{y}/SLC-1(-/-) mice, but the KK/SLC-1(-/-) mice exhibited significantly increased eating, compared with the KK/SLC-1(+/+) mice (FIG. 6B) .
In plasma glucose level, there was no difference between the KKA^{y}/SLC-1(+/+) mice and the KKA^{y}/SLC-1(-/-) mice at 5 weeks of age (FIG. 7A). Beyond 9 weeks of age, the KKA^{y}/SLC-1(+/+) mice exhibited hyperglycemia, but in the KKA^{y}/SLC-1(-/-) mice, the elevation in plasma glucose level was suppressed (P<0.05, at 16 weeks of age) (FIG. 7A). On the other hand, in the KK/SLC-1(+/+) mice and the KK/SLC-1(-/-) mice, no elevation in plasma glucose level due to aging was observed, and there was no difference between the two types of mice (FIG. 7A).
The plasma triglyceride level was significantly lower because of SLC-1 deficiency both in the KKA^{y} mice and in the KK mice (P<0.05, at 16 weeks of age) (FIG. 7B).
The plasma insulin level tended to decrease because of SLC-1 deficiency both in the KKA^{y} mice and in the KK mice (FIG. 7C).
Regarding plasma adiponectin levels, the KKA^{y}/SLC-1(-/-) mice at 5 weeks of age had lower values compared with the KKA^{y}/SLC-1(+/+) mice (P<0.05) (FIG. 7D). Beyond 9 weeks of age, the plasma adiponectin level decreased in the KKA^{y}/SLC-1(+/+) mice but increased in the KKA^{y}/SLC-1(-/-) mice (P<0.01, at 16 weeks of age) (FIG. 7D). On the other hand, in the KK/SLC-1(+/+) mice and the KK/SLC-1(-/-) mice, the plasma adiponectin level increased with aging, and no difference was observed between the two groups (FIG. 7D).
The plasma leptin level was elevated with aging in the KKA^{y}/SLC-1(+/+) mice. The KKA^{y}/SLC-1(-/-) mice, compared with the KKA^{y}/SLC-1(+/+) mice, exhibited significantly lower values until 9 weeks of age (P<0.05, at 9 weeks of age), but thereafter no difference was observed with aging (FIG. 7E). On the other hand, the plasma leptin level was elevated with aging in the KK/SLC-1(+/+) mice, but lower values were maintained in the KK/SLC-1(-/-) mice (P<0.01, at 16 weeks of age) (FIG. 7E).
The HbA1c level was significantly lower in the KKA^{y}/SLC-1(-/-) mice, compared with the KKA^{y}/SLC-1(+/+) mice, which exhibited higher values in reflection of the diabetic state (FIG. 7F). In the KK/SLC-1(+/+) mice and the KK/SLC-1(-/-) mice, normal values were obtained (FIG. 7F).
The plasma NEFA level at 18 weeks of age was significantly lower in the KKA^{y}/SLC-1(-/-) mice, compared with the KKA^{y}/SLC-1(+/+) mice, and there was the same tendency for the KK/SLC-1(+/+) mice and the KK/SLC-1(-/-) mice (FIG. 7G).
The plasma corticosterone level at 21 weeks of age was higher in the KKA^{y}/SLC-1(+/+) mice, and significantly lower in the KKA^{y}/SLC-1(-/-) mice, compared with the KKA^{y}/SLC-1(+/+) (FIG. 7H). No difference was observed between the KK/SLC-1(+/+) mice and the KK/SLC-1(-/-) mice.
In plasma total T4 level, no difference was observed among the four groups (FIG. 7I).

### Example 9 Body fat percentages in KKA^{y} mouse and KK mouse hybrid groups

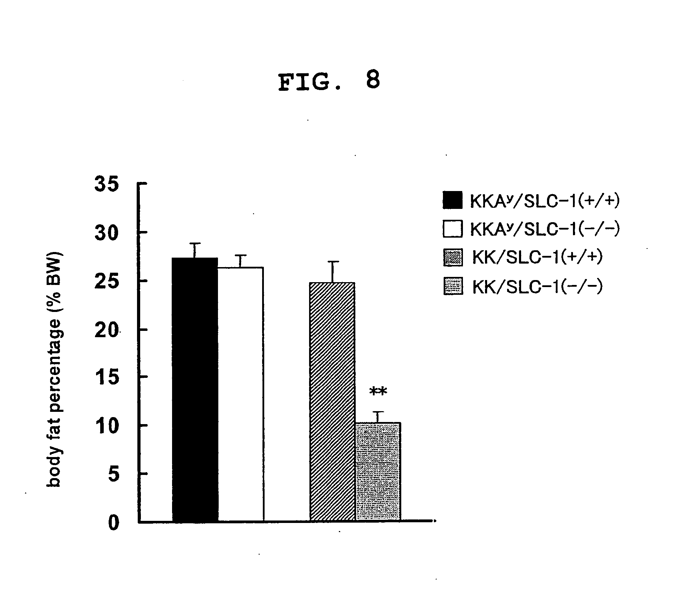

Body fat percentages in mice at 17 weeks of age were measured in the same manner as Example 4. As a result, no difference was observed in the body fat percentage in KKA^{y}/SLC-1(-/-) mice, compared with KKA^{y}/SLC-1(+/+) mice, but the body fat percentage in KK/SLC-1(-/-) mice was significantly lower than that in KK/SLC-1(+/+) mice (FIG. 8).

### Example 10 Energy consumption by KKA^{y} mouse and KK mouse hybrid groups

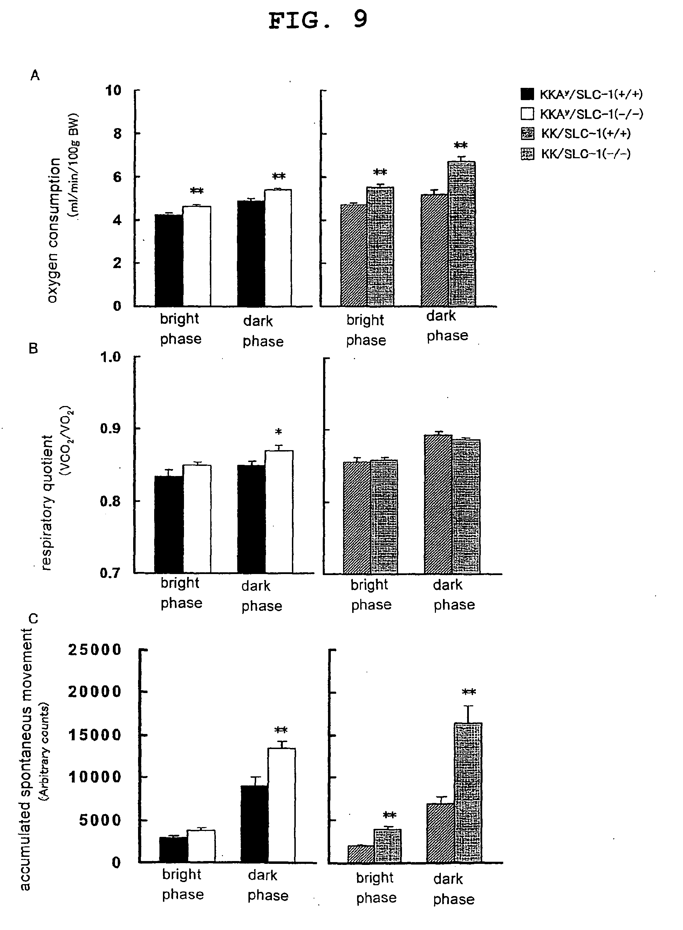

Oxygen consumption and spontaneous movement were measured in the same manner as Example 5.
Oxygen consumption in the maturity stage (13 to 14 weeks of age) increased significantly because of SLC-1 deficiency throughout the bright phase and the dark phase, both in KKA^{y} mice and KK mice, compared with respective control mice (FIG. 9A). This phenomenon was also observed in the young stage (5 to 6 weeks of age) (data not published). The respiratory quotient became higher in the dark phase in KKA^{y}/SLC-1(-/-) mice in the maturity stage, compared with control mice, suggesting accentuation of the consumption of carbohydrates (FIG. 9B). Spontaneous movement at 7 to 9 weeks of age increased, compared with respective control mice, in the KKA^{y}/SLC-1(-/-) mice and KK/SLC-1(-/-) mice (FIG. 9C).

### Example 11 Glucose tolerance test on KKA^{y} mouse and KK mouse hybrid groups

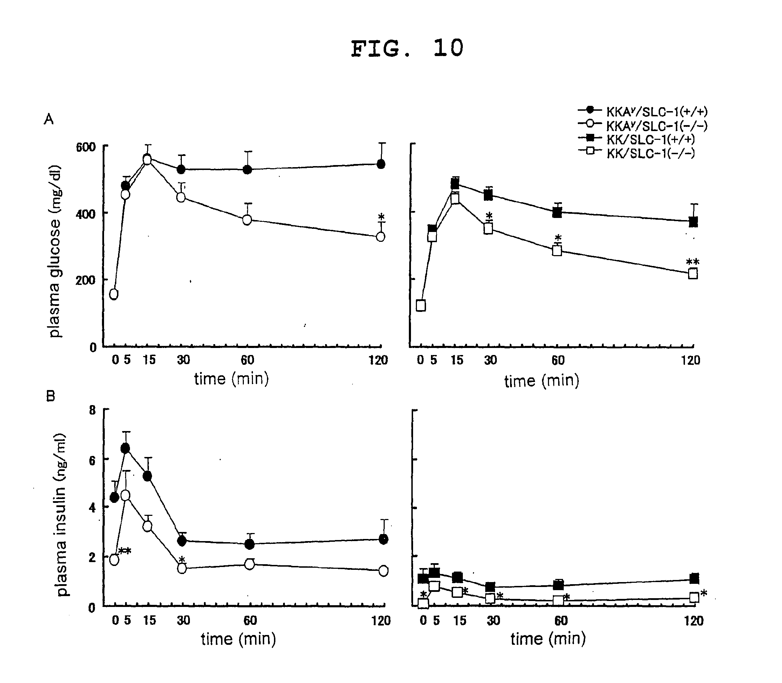

A glucose tolerance test was performed on mice at 16 weeks of age in the same manner as Example 6.
For blood parameters for the KKA^{y} group during satiation before the glucose tolerance test, KKA^{y}/SLC-1(-/-) mice, compared with KKA^{y}/SLC-1(+/+) mice, showed no change in body weight or plasma leptin level, but showed a significant reduction (32.5%) in plasma glucose level (FIGs. 6A, 7A, 7E). For plasma glucose levels during the glucose tolerance test, no difference was observed between the two types of mice during fasting (0 minutes before glucose loading), but the KKA^{y}/SLC-1(-/-) mice exhibited a lower value beyond 30 minutes after the glucose loading (FIG. 10A). When AUC (area under the glucose curve, 0 to 120 minutes) was calculated, the KKA^{y}/SLC-1(-/-) mice showed a 24.0% reduction, compared with the KKA^{y}/SLC-1(+/+) mice. The plasma insulin level in the KKA^{y}/SLC-1(-/-) mice was significant lower during fasting (0 minutes before glucose loading) and 30 minutes after the glucose loading (FIG. 10B).
In the KK group, no difference in body weight and plasma glucose level during satiation was observed between the KK/SLC-1(+/+) mice and the KK/SLC-1(-/-) mice, but the plasma leptin level in the KK/SLC-1(-/-) mice decreased significantly by 76.6% (P<0.01), and the plasma insulin level showed a reduction of 91.1% (FIGs. 6A, 7A, 7C, 7E). In the glucose tolerance test, plasma glucose level was significantly lower in the KK/SLC-1(-/-) mice at 30, 60, and 120 minutes after glucose loading (FIG. 10A), AUC (0 to 120 minutes) decreased significantly by 26.2% (P<0.01), and the plasma insulin level showed significantly lower values during fasting and beyond 15 minutes after glucose loading (FIG. 10B). These results for the KKA^{y} group and the KK group were similar to those obtained at 6 weeks of age prior to the onset of obesity and diabetes (data not published).

### Example 12 Gene expression in tissues from KKA^{y} mouse and KK mouse hybrid groups

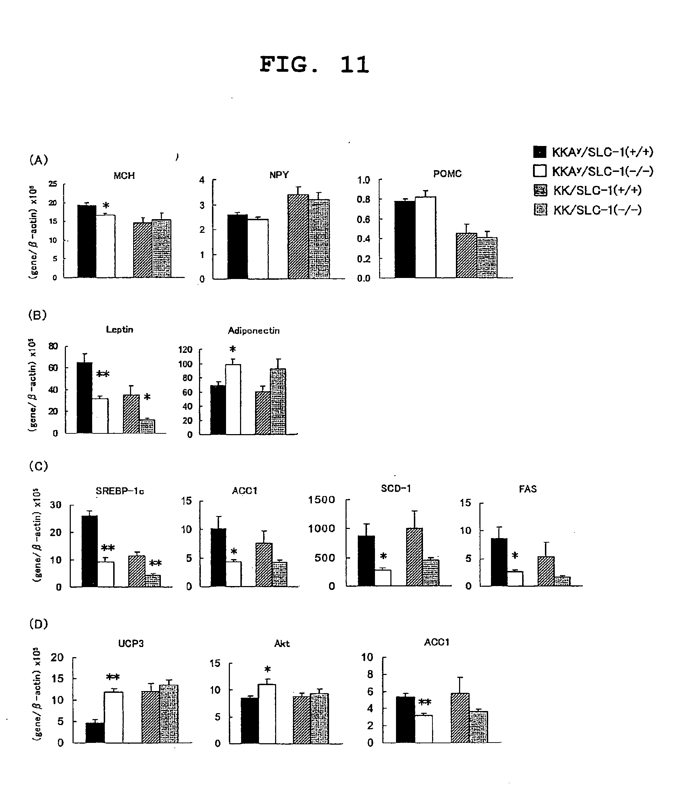

For KKA^{y} mouse and KK mouse hybrid groups, changes in the expression of feeding-related genes and metabolism-related genes in the initial stage of the onset of diabetes were examined by real time PCR.
From five mice in each group at 9 weeks of age, the diencephalon, perigenital white adipose tissue, liver, and skeletal muscle were collected, and total RNA was extracted with ISOGEN (produced by Nippon Gene), after which it was purified using the RNeasy mini kit (produced by QIAGEN). The total RNA was reverse-transcribed using TaqMan Reverse Transcription Reagents (produced by Applied Biosystems Japan), and this was used as the template for expression level analysis by real time PCR (ABI7700, produced by Applied Biosystems Japan). The measured values were corrected by the value for the β-actin gene.
In the diencephalon, in KKA^{y}/SLC-1(+/+) mice, the expression of the gene for melanin-concentrating hormone (MCH), which is an endogenous ligand, increased (31.3%, P<0.05), compared with KK/SLC-1(+/+) mice, but this was suppressed to the same extent as the KK mice because of SLC-1 deficiency (FIG. 11A). No difference was observed between the KK/SLC-1(+/+) mice and KK/SLC-1(-/-) mice. In the KKA^{y} mice, decreased expression of the neuropeptide Y (NPY) gene and increased expression of the proopiomelanocortine (POMC) gene were observed, compared with the KK mice, but no influence of SLC-1 deficiency was observed in the gene expression in either type of mice (FIG. 11A). Regarding the expression of the agouti-related peptide (AgRP) gene, ghrelin gene, and corticotropin-releasing hormone (CRH) gene, no difference was observed among the four groups (data not published).
In perigenital white adipose tissue, the KKA^{y}/SLC-1(-/-) mice, compared with the KKA^{y}/SLC-1(+/+) mice, exhibited significantly decreased expression of the gene for leptin, which is secreted from fat cells and acts negatively on eating regulation and energy consumption, and significantly increased expression of the gene for adiponectin, which is involved in obesity, diabetes, and arteriosclerosis suppression (FIG. 11B). The expression of the gene for tumor necrosis factor alpha (TNF-α), which is involved in insulin resistance, decreased by 41.1%, but the difference was insignificant (data not published). The same influence of SLC-1 deficiency was observed in the KK mice as in the KKA^{y} mice (FIG. 11B), but additionally the expression level of the gene for peroxisome proliferator-activated receptor-gamma (PPARy), which is involved in lipid metabolism and glucose metabolism, increased by 124.2% (P<0.05) because of deficiency of the SLC-1 gene (data not published).
In the liver, in the KKA^{y} mice, compared with the KK mice, the expression of the sterol regulatory element binding protein-1c (SREBP-1c) gene, acetyl-CoA carboxylase 1 (ACC1) gene, and fatty acid synthase (FAS) gene, which are involved in lipid metabolism, tended to increase. In the KKA^{y}/SLC-1(-/- ) mice, compared with the KKA^{y}/SLC-1(+/+) mice, the expression of the SREBP-1c gene, ACC1 gene, FAS gene, and stearoyl CoA desaturase 1 (SCD-1) gene decreased (FIG. 11C). In the KK/SLC-1(-/-) mice, the same tendency as with the KKA^{y} group was observed.
In skeletal muscle, in the KKA^{y}/SLC-1(-/-) mice, compared with the KKA^{y}/SLC-1(+/+) , the expression of the uncoupling protein 3 (UCP3) gene and the Akt kinase gene, which is involved in glucose metabolism, increased, and the expression of the ACC1 gene decreased (FIG. 11D).

### [Industrial Applicability]

The non-human mammal deficient in the expression of the SLC-1 gene of the present invention is useful in the analysis of the functions of SLC-1 and the like. The obesity and/or type II diabetes model non-human mammal that is deficient in the expression of the SLC-1 gene of the present invention is useful in the development of a prophylactic/therapeutic drug for obesity and/or type II diabetes and the like. According to the present invention, SLC-1 antagonists are capable of promoting adiponectin production; it is suggested that they may be useful as pharmaceuticals for diabetes accompanied by obesity and the like.

While the present invention has been described with emphasis on preferred embodiments, it is obvious to those skilled in the art that the preferred embodiments can be modified. The present invention intends that the present invention can be embodied by methods other than those described in detail in the present specification. Accordingly, the present invention encompasses all modifications encompassed in the gist and scope of the appended "CLAIMS."
This application is based on a patent application No. 2006-176978 filed in Japan (filing date: June 27, 2006), the contents of which are incorporated in full herein by this reference. In addition, the contents disclosed in any publication cited herein, including patents and patent applications, are hereby incorporated in their entireties by reference, to the extent that they have been disclosed herein.

## Claims

1. A non-human mammal deficient in the expression of the SLC-1 gene, having the following characteristics:
(1) a lower blood insulin level in glucose tolerance test,
(2) increased insulin sensitivity,
(3) higher resistance to obesity even on high fat diet,
(4) a smaller white fat cell size, and
(5) accentuated lipolysis
compared with the corresponding wild-type animal, or a portion of the body thereof.

2. The animal of claim 1, further having the following characteristics:
(i) accentuated spontaneous movement and oxygen consumption,
(ii) decreased body fat, and
(iii) a decreased plasma leptin level
compared with the corresponding wild-type animal, or a portion of the body thereof.

3. The animal of claim 1, wherein the non-human mammal is a mouse or a rat, or a portion of the body thereof.

4. An obesity and/or type II diabetes model non-human mammal that is deficient in the expression of the SLC-1 gene, having the following characteristics:
(1) elevated adiponectin expression,
(2) delayed onset of hyperglycemia,
(3) a lower blood glycohemoglobin level, and
(4) accentuated energy consumption
compared with the corresponding obesity and/or type II diabetes model non-human mammal wherein the expression of the gene is normal, or a portion of the body thereof.

5. The animal of claim 4, further having the following characteristics:
(i) increased oxygen consumption, and
(ii) a decreased blood corticosterone level
compared with the corresponding obesity and/or type II diabetes model non-human mammal wherein the expression of the SLC-1 gene is normal, or a portion of the body thereof.

6. The animal of claim 4, wherein the non-human mammal is a mouse or a rat, or a portion of the body thereof.

7. The animal of claim 6, wherein the obesity and/or type II diabetes model non-human mammal is a KKA^{y} mouse, or a portion of the body thereof.

8. A promoter of adiponectin production comprising an SLC-1 antagonist.

9. The agent of claim 8, which is administered to a patient with metabolic syndrome or arteriosclerotic disease accompanied by a decreased adiponectin level.

10. A method of promoting adiponectin production, comprising antagonistically inhibiting SLC-1.

11. A use of an SLC-1 antagonist for producing a promoter of adiponectin production.
